# EUROPÄISCHE PATENTSCHRIFT

(11) **EP 4 355 385 B1**
(45) Veröffentlichungstag und Bekanntmachung des Hinweises auf die Patenterteilung: **15.04.2026**
(21) Anmeldenummer: 22734907.3
(22) Anmeldetag: 14.06.2022
(51) Int. Cl.: A61M 5/142, A61M 5/145, H02N 2/02

(54) **PIEZO-AKTUIERTE ANTRIEBSEINHEIT FÜR SPRITZENPUMPE**
PIEZO-ACTUATED DRIVE UNIT FOR SYRINGE PUMP
UNITÉ D'ENTRAÎNEMENT PIÉZO-ACTIONNÉE POUR POMPE À SERINGUE

(30) Priorität: 18.06.2021 DE 102021115809
(43) Veröffentlichungstag der Anmeldung: 24.04.2024
(73) Patentinhaber: B. Braun Melsungen AG, 34212 Melsungen (DE)
(72) Erfinder: STICH, Marcel, 34329 Nieste (DE)
(74) Vertreter: Winter, Brandl - Partnerschaft mbB
(86) Internationale Anmeldenummer: PCT/EP2022/066121
(87) Internationale Veröffentlichungsnummer: WO 2022/263417

(56) Entgegenhaltungen:
- US-A- 4 736 131
- US-A- 5 319 257
- US-A- 6 104 125
- US-A1- 2011 152 827

## Beschreibung

### Technisches Gebiet

Die vorliegende Offenbarung betrifft eine piezoelektrische Antriebseinheit für eine medizintechnische Verwendung, insbesondere zum Einbau in einer medizintechnischen Spritzenpumpe, Infusionspumpe, Dosierpumpe und dergleichen. Ferner wird ein zugehöriges Verfahren zum Erzeugen eines Schlittenvorschubs des Schlittens der piezoelektrischen Antriebseinheit offenbart. Zudem werden eine zugehörige medizintechnische Spritzenpumpe mit der piezoelektrischen Antriebseinheit sowie ein dieser zugehöriges Bauteil vorgeschlagen. Ferner wird ein Computerprogramm auf einem Datenträger zur Ausführung des Verfahrens durch eine Steuereinheit der offenbarungsgemäßen piezoelektrischen Antriebseinheit vorgeschlagen.

### Stand der Technik

In der Medizintechnik kommen vielfach Spritzenpumpen bzw. Infusionspumpen zur präzisen Förderung bzw. Dosierung von medizinischen Flüssigkeiten wie flüssig vorgelegten bzw. zubereiteten Medikamenten, Wirkstofflösungen, Infusionen und dergleichen zum Einsatz. Dabei erfolgt insbesondere die Förderung bzw. Dosierung, indem eine in die Spritzenpumpe eingesetzte, die Wirkstofflösung beinhaltende Spritze kontrolliert ausgedrückt wird. Ein Beispiel einer im Markt befindlichen Spritzenpumpe ist unter dem Handelsnamen "Space plus Perfusor" der Firma B. Braun bekannt. Zur präzisen Förderung bzw. Dosierung bewirkt eine in der Spritzenpumpe verbaute Antriebseinheit eine Linearbewegung eines zugehörigen Spritzenkolbens. Diese Linearbewegung zur Förderung bzw. Dosierung wird konstruktiv in der Regel mittels Spindelantrieben sowie Untersetzungsgetrieben zum Vorschub eines auf den Spritzenkolben wirkenden Kolbenstößels der Antriebseinheit erreicht. Jedoch ist hinsichtlich der Bauweise derartiger Antriebssysteme nachteilig, dass die Anzahl beweglicher, zueinander zu passender Teile hoch ausfällt, was vermehrte Verschleißstellen sowie Fehlerquellen bedingt. Dadurch sind wichtige Aspekte solcher Systeme nachteilig limitiert, was insbesondere deren Genauigkeit, Langlebigkeit, Robustheit, Wartungserfordernisse anbelangt sowie Herstellkosten betrifft.

Ferner ist im Bereich der Antriebstechnik von Dosier- bzw. Präzisonspumpen die Verwendung von piezoelektrischen Elementen (Einheiten) bzw. Piezo-Aktoren (Piezo-Aktuatoren; kurz: Piezos) grundsätzlich bekannt, insbesondere zur Ausbildung von piezoelektrischen Stellantrieben, jedoch auch anderweitig. Beispielsweise betrifft WO 2016/153171 A1 eine Abgabepumpe in einem Verfahren zur Herstellung von Elektronik mit einem Linearantrieb zur direkten Ventildosierung einer Flüssigkeit wie einem Kunstharz, welche mittels Betätigung einer Ventilstange aus einem Vorhaltereservoir in einem Ventilkörper über eine Düse direkt dosiert wird. Dabei wird mittels der periodischen Ausdehnung und linearen Verformung einer piezoelektrischen Einheit eine Führungsstange des Linearantriebs, entlang welcher sich ein an der Ventilstange angeordnetes bewegliches Element bewegen kann, zur direkten Ventildosierung linear bewegt.

Darüberhinaus ist in JP 2006 281178 A eine insbesondere miniaturisierbare Spritzenpumpe für eine Produktionslinie in der Halbleiterfertigung offenbart, die in der Lage ist, eine winzige Menge, bspw. 0,1 Mikroliter, selbst einer viskosen Flüssigkeit, wie beispielsweise eines Klebstoffs oder einer Paste, mit hoher Geschwindigkeit hochpräzise abzulassen. Dazu kann aufgrund der Expansion und Kontraktion eines piezoelektrischen Elements eine mit einer Antriebsstange versehene bewegliche Vorrichtung eines Kolbens um eine winzige Menge in Förderrichtung genau bewegt werden. Wenn sich dabei die Antriebsstange zum Vorschub in eine erste Richtung bewegt, wird eine drehbar gelagerte Kugel eines Kupplungselements zu dessen Verriegelung in Druckkontakt gebracht. Dabei bewirkt die Verriegelung, dass die Bewegung des Kupplungselements gesperrt ist, wenn sich die Antriebsstange in eine zweite Richtung bewegt. Bei Verwendung einer solchen an einem Roboterarm angebrachten Spritzenpumpe können dessen Bewegungen mit einer hohen Geschwindigkeit sowie Beschleunigung realisiert werden, was die Taktzeit der Produktionslinie verkürzt. Insofern die hierin vorgeschlagene Lösung das Kupplungselement mit der drehbar an einer schrägen Oberfläche gelagerten Kugel sowie Schieber- und Federelemente umfasst, ist diese dennoch in nachteiliger Weise spielbehaftet bzw. erfordert ein komplexes Zusammenspiel von einzelnen Passungen der Bauteile zueinander. Zusätzlich nachteilig hinsichtlich einer Verschleißproblematik ist, dass bei dem die Verriegelung bewirkenden Druckkontakt der drehbar gelagerten Kugel sehr hohe, nämlich punktförmig wirkende, Kräfte bzw. Belastungsspitzen der Flächenpressung (vgl. Hertz'sche Pressung bei Kontakt gekrümmter Oberflächen) in dem Kupplungselement auftreten. Des Weiteren fällt aufgrund des geringen Hubs bzw. der geringen Dosiermenge eine leistbare Förderrate entsprechend niedrig aus.

Weiter offenbart CN 102192135 eine piezoelektrische Pumpe, welche auf dem Grundprinzip einer Verdrängungspumpe in Abwandlung einer, als solche für die Medizintechnik bekannten, Membranpumpe beruht. Dabei wird die Hubbewegung der Membran nicht in üblicher Weise mittels eines Drehmotors, sondern mittels eines Piezos erzeugt.

Wiederum sind aus der EP 2198164 B1 sowie der EP 3337534 A1 medizintechnische Spritzenpumpen(antriebe) bekannt, bei welchen Piezoelektrizität zur Erzeugung von Rotation bzw. einem Drehmoment in einem ansonsten herkömmlichen uni-direktionalen Spindelantrieb (wie eingangs beschrieben) genutzt wird. Beispielsweise offenbart EP 3337534 A1 einen Antriebsmechanismus für eine tragbare piezoelektrische (Mikrofluid-)Pumpe zur Arzneimittelabgabe aus einer, in einer Patrone vorgesehenen,Spritze an einen Patienten. In der Pumpe verursachen dazu piezoelektrische Elemente beim Erregen eine lineare Bewegung einer Schubstange, die mit der Spritze in Verbindung steht, um die Spritze voranzutreiben und das Medikament abzugeben. Dabei wird das hohe Drehmoment, das von den piezoelektrischen Elementen erzeugt wird, direkt in das gleiche Drehmoment an der Leitspindel umgewandelt, so dass kein drehmomenterhöhendes Untersetzungssystem erforderlich ist.

US 4 736 131 A offenbart eine Linearmotor-Antriebsvorrichtung mit einem Paar von Aktuatoren, die jeweils ein erstes piezoelektrisches Element aufweisen, ein Paar Hebel, die mit dem ersten piezoelektrischen Element verbunden sind, und eine Vorrichtung zum Vergrößern der durch das erste piezoelektrische Element verursachten Verschiebung. Ein zweites piezoelektrisches Element ist mit dem Paar der Aktuatoren verbunden. Das zweite piezoelektrische Element verschiebt die Hebel in eine Richtung senkrecht zur Betätigungsrichtung der Hebel.

US 6 104 125 A offenbart einen linearen Aktuator mit zwei beabstandeten Sitzen, die durch ein zentrales piezoelektrisches Element verbunden sind. Die Sitze sind in einem Schienenrahmen montiert. Jeder Sitz hat ein piezoelektrisches Element und eine elastische Zone zum Eingreifen oder Lösen mit dem Schienenrahmen. Durch abwechselndes Erregen und Abschalten des mittleren piezoelektrischen Elements kann das mittlere piezoelektrische Element abwechselnd ausgefahren oder zusammengezogen werden und bewegt somit die Sitze und folglich den Aktuator in Längsrichtung entlang des Schienenrahmens. Somit kann eine lineare und genaue Bewegung des Aktuators auf Mikrometerebene erreicht werden.

US 5 319 257 A offenbart ein einachsiges Antriebssystem oder einen Mikroaktuator, das/der in einer Ultrahochvakuumumgebung betrieben werden kann. Der Mechanismus umfasst eine flexible Kupplung mit einer durchgehenden Bohrung und zwei Klemm-/Schieberanordnungen, die an axialen Enden der Kupplung angebracht sind. Die Klemm/Schieber-Anordnungen werden durch darin befindliche spannungsbetriebene Piezoelektrika erregt, um mit der Welle und der Kupplung betriebsmäßig in Eingriff zu kommen, wodurch bewirkt wird, dass sich die Welle entlang ihrer Rotationsachse durch die Bohrung bewegt.

US 2011/152827 A1 offenbart einen Pumpenantrieb zur Arzneimittelabgabe, der einen linearen piezoelektrischen Motor verwendet, um einen Spritzenkolben vorzuschieben, um ein flüssiges Arzneimittel abzugeben. Dabei fungiert die Mechanik auf einem Bauprinzip auf Basis einer Leitspindel mit einer Rotationsachse und einer Ratsche.

Im vorstehend genannten Stand der Technik bestehen somit eine Reihe von Nachteilen bzw. konstruktiv bedingten Limitationen. Insbesondere besteht bei den medizintechnischen Spritzenpumpen in der Bauweise mit (piezoelektrischem) Spindelantrieb (weiterhin) das Problem, dass diese spielbehaftet ausfallen. Insofern benötigen Spindelantriebe viele ineinander eingreifende, bewegliche Bauteile (Spindel, Zahnräder, Motor, Lagerung, etc.). Um also bei dieser Bauweise eine gewünschte bzw. erforderliche Genauigkeit bzw. Präzision der Förderung bzw. Dosierung der medizinischen Flüssigkeit zu erreichen, müssen sehr enge Maßtoleranzen bzw. Passungen gewählt werden. Dabei müssen die einzelnen Maßtoleranzen bzw. Passungen aufeinander in der Konstruktion abgestimmt und entsprechend nach deren Fertigung in der Qualitätskontrolle aufwendig verifiziert bzw. sichergestellt werden. Des Weiteren geht mit beweglichen Bauteilen Verschleiß einher; aufgrunddessen zieht dies einen mittelfristigen Genauigkeitsverlust bzw. aufwendige Wartungserfordernisse nach sich.

Ferner bedingt die vorstehend genannte Bauweise mit (piezoelektrischem) Spindelantrieb nachteilig einen erhöhten Bauraumbedarf, welcher trotz eines geringen Hubs bzw. trotz einer entsprechend geringen Förderrate (bzw. Pumpleistung) besteht. Zusätzlich zeigen sich im Betrieb weitere Nachteile eines erhöhten Energieverbrauchs, des Entstehen von Abwärme bzw. von betriebsbedingten Geräuschen. Ferner ist bei Schreitantrieben und Schwingantrieben eine geringe Haltekraft als nachteilig zu verzeichnen. Demzufolge bietet die herkömmliche Bauweise nur eine beschränkte Eignung zum Einsatz bei mobilen Patientenvorrichtungen wie tragbaren Infusionspumpen.

Somit besteht ein Bedarf, die vorstehend genannten Gesichtspunkte zu verbessern und die Nachteile im Stand der Technik zu überwinden. Eine Aufgabe, die durch die vorliegende Offenbarung gelöst werden soll, besteht darin, eine leisere, genauere und stabilere Antriebseinheit zur Verwendung in medizintechnischen Dosier- bzw. Präzisionspumpen, insbesondere für Infusionspumpen, bereitzustellen. Dabei sollen Einstell- und Kalibrieraufwände reduziert werden. Insbesondere soll sich die Antriebseinheit beim Einsatz in einer medizintechnischen Spritzenpumpe dazu eignen, über längere Zeiträume eine gleichmäßige, präzise Dosierung, insb. Medikamentenabgabe, zu leisten. Ferner gilt es, eine Bauweise für eine Antriebseinheit darzustellen, die trotz hoher Kräfte eine geringere oder sogar gar keine Anfälligkeit zum Verschleiß aufweist. Weitere Aufgaben bestehen darin, die Antriebseinheit und damit die Gesamtpumpe leichter zu gestalten sowie die Energieeffizienz zu erhöhen.

Die Aufgabe der Offenbarung wird mit einer Piezo-aktuierten bzw. piezoelektrischen Antriebseinheit für eine medizintechnische (Spritzen-)Pumpe mit den Merkmalen des Anspruchs 1 gelöst.

Gemäß der Offenbarung (bzw. gemäß einem erstgenannten Aspekt der vorliegenden Offenbarung) weist eine piezoelektrische Antriebseinheit auf (bzw. hat): einen Antriebsrahmen, eine Steuereinheit, eine Schubstange, eine Linearführung, einen Schlitten und eine erste Klemmvorrichtung des Schlittens. Dabei ist die piezoelektrische Antriebseinheit zum Abgeben und/oder Dosieren medizinischer Flüssigkeiten aus einem medizinischen Behälter eingerichtet. Insbesondere ist die piezoelektrische Antriebseinheit für eine (insb. zum Einbau in eine) in einer axialen Längsrichtung verfahrende medizintechnische Spritzenpumpe, Infusionspumpe oder dergleichen eingerichtet. Dabei ist die Schubstange an dem Antriebsrahmen mittels einer Längshub-Vorrichtung in der Längsrichtung um einen Längshub oszillierend bewegbar eingerichtet, um bei Einwirken zumindest eines ersten Piezo-Aktors der Längshub-Vorrichtung durch dessen (aktuiertes) Ausdehnen und Zusammenziehen vor- und zurückbewegt zu werden.

Der zumindest eine erste Piezo-Aktor ist mit der Steuereinheit verbunden. Sprich, die Steuereinheit ist eingerichtet, den zumindest einen ersten Piezo-Aktor zu aktuieren, um um einen ersten Piezohub zu oszillieren bzw. sich auszudehnen und zusammenzuziehen. Dabei ist die Linearführung an dem Antriebsrahmen (im Wesentlichen) parallel zu der Schubstange angeordnet. Dabei ist der Schlitten zur Förderung in der Längsrichtung entlang der Linearführung verfahrbar. Der Begriff Förderung meint insbesondere einen Förderantrieb einer medizintechnischen (Spritzen-) Pumpe mittels der offenbarungsgemäßen piezoelektrischen Antriebseinheit, wobei diese insbesondere in der (Spritzen-) Pumpe (insb. fest) verbaubar bzw. verbaut sein kann.

Ferner ist dabei die Steuereinheit offenbarungsgemäß eingerichtet, die erste Klemmvorrichtung des Schlittens zu koordinieren (bzw. anzusteuern). Die erste Klemmvorrichtung ist eingerichtet (bzw. wird angesteuert), um zwischen einem ersten Kopplungszustand zur Kopplung des Schlittens an der Schubstange und einem ersten Entkopplungszustand zur Entkopplung des Schlittens von der Schubstange zu wechseln. Der erste Kopplungszustand ist vorgesehen, um während des ersten Kopplungszustandes einen Schlittenvorschub des Schlittens gemäß dem Längshub der von einer axialen Ausgangsposition bewegbaren Schubstange zu erzeugen. Der erste Entkopplungszustand ist vorgesehen, um die Schubstange für eine von dem Schlitten getrennte Bewegung freizugeben, insbesondere für eine Rückzugsbewegung zurück auf die Ausgangsposition freizugeben.

Offenbarungsgemäß ist dabei die erste Klemmvorrichtung eingerichtet, zumindest einen zu ihrer Aktuierung (bzw. Betätigung, Auslösung, piezoelektrisch aktuierter Oszillation) mit der Steuereinheit verbundenen zweiten Piezo-Aktor aufzuweisen. Dabei ist der zumindest eine zweite Piezo-Aktor eingerichtet, durch dessen Ausdehnen oder Zusammenziehen zumindest den ersten Kopplungszustand zu bewirken. Sprich, die Steuereinheit ist eingerichtet, den zumindest einen zweiten Piezo-Aktor zu aktuieren, um um einen zweiten Piezohub zu oszillieren bzw. sich auszudehnen und zusammenzuziehen.

Der Haltemechanismus der ersten Klemmvorrichtung funktioniert, indem die (zunächst) vorgeschobenen Schubstange (sodann) festgehalten wird. Der Haltemechanismus der ersten Klemmvorrichtung ist ausreichend für den Fall, dass nicht noch weitere Zugkräfte an dem Schlitten angreifen, bzw. ist insbesondere hinreichend, um lastfrei den Schlitten hin und her zu schieben. Dabei meint der Begriff des Schlittens den entlang der Schubstange axial beweglichen bzw. verfahrbaren (tragenden) Grundkörper.

Die erste Klemmvorrichtung zeichnet sich gegenüber dem Stand der Technik besonders aufgrund des Vorteils aus, dass die zwischen dem Schlitten und der Schubstange (in einer Querrichtung zu der Längsrichtung) aufzubringende bzw. aufbringbare erste Klemmkraft direkt , d.h. ohne (komplexe) Kraftumlenkung, bewirkbar ist.

Dabei bezeichnet der Begriff des Piezo-Aktors (bzw. Piezo-Aktuators) ein (piezoelektrisches) Bauteil, das den (inversen) Piezoeffekt ausnutzt, um durch Anlegen einer elektrischen Spannung eine mechanische Bewegung bzw. Auslenkung (definiert) auszuführen. Wenn an den Piezo-Aktor die elektrische Spannung (über eine Steuereinheit) angelegt wird, erfolgt als die Auslenkung eine Bewegungsamplitude bzw. eine (relative) Längsausdehnung bzw. ein sogenannter Piezohub des Piezo-Aktors im Bereich von Mikrometern; dabei zieht sich dieser in Querrichtung zusammen. Die Auslenkung bleibt so lange bestehen, wie die elektrische Spannung angelegt bleibt. Aufgrunddessen betrifft die Bewegungsamplitude bzw. (relative) Längsausdehnung des Piezo-Aktors eine reversible bzw. oszillierende Auslenkung.

Bei Verwendung des Piezo-Aktors ist die Bewegungsamplitude bzw. die relative Längsausdehnung des Piezo-Aktors vorzugsweise steuerbar, insbesondere durch Anlegen der elektrischen Spannung steuerbar. Insofern ist die Bewegungsamplitude bzw. die (relative) Längsausdehnung proportional zur elektrischen Feldstärke (wobei die Feldstärke als Quotient Spannung/Elektrodenabstand definiert ist). Insbesondere ist die Bewegungsamplitude bzw. die relative Längsausdehnung, d.h. eine Ist-Größe bzw. ein aktueller Piezohub derselben, in Abhängigkeit der elektrischen Spannung steuerbar. Sprich, der Piezo-Aktor ist eingerichtet, die Bewegungsamplitude bzw. die relative Längsausdehnung als einen vollständigen oder teilweisen Anteil eines nominalen bzw. theoretischen bzw. maximalen Wertes der (relativen) Auslenkung bzw. Längsausdehnung des Piezo-Aktors abzubilden bzw. auszuführen.

Vorzugsweise kann der Piezo-Aktor als ein mehrlagiges Piezoelement (bzw. Multilayer-Element, Piezostapel, engl.: "piezo stack") ausgeführt sein. Dazu werden mehrere dünne, insbesondere keramische, Piezoelemente mit dazwischenliegenden Elektroden zu einer Reihenanordnung bzw. in Sandwichbauweise zusammengefügt.

Dabei kann die Steuereinheit zur Steuerung der piezoelektrischen Antriebseinheit mit einer Speichereinheit bzw. einem Datenträger (bzw. maschinenlesbaren Speichermedium) ausgebildet oder ausrüstbar sein. Vorzugsweise kann die Speichereinheit bzw. der Datenträger fest integrierbar oder integriert sein. Alternativ oder kumulativ kann die Steuereinheit über eine Schnittstelle mit einer hinsichtlich der Steuereinheit externen Speichereinheit elektrisch (bzw. signaltechnisch) verbunden oder verbindbar sein.

Gemäß der vorliegenden Offenbarung ergeben sich mehrere Vorteile bzw. Verbesserungen gegenüber dem Stand der Technik. So zeichnet sich die offenbarungsgemäße piezoelektrische Antriebseinheit durch eine hohe Genauigkeit bei gleichzeitig hohen Kräften zur Förderung bzw. zum präzisen, sicheren Verfahren und Führen im Antrieb aus. Insofern wird eine besonders einfache und schnelle, dennoch gleichzeitig sensitive Rezirkulationsmessung vorgeschlagen. Durch die offenbarungsgemäß gesamthaft eingesetzte Piezotechnik reduziert sich gegenüber dem Stand der Technik vorteilhaft die Anzahl bewegter Bauteile, womit ein verminderter Verschleiß bzw. eine erhöhte Robustheit einhergehen. Zudem resultiert dies in äußerst günstiger Weise in einem verringerten Bauraum der Antriebseinheit. Gleichzeitig gelingt es, diese erheblich vorteilhaften Optimierungen bei mindestens beibehaltener oder sogar höherer Leistung sowie Genauigkeit der vorgeschlagenen Antriebseinheit zu erzielen.

Als noch weiterer Vorteil gegenüber herkömmlichen Lösungen ist zu nennen, dass die die offenbarungsgemäße piezoelektrische Antriebseinheit eine(n) bi-direktionale(n) Antriebsweise ermöglicht. Insofern ist ein in der Längsrichtung positiver wie negativer Schlittenvorschub realisierbar bzw. verfahrbar.

Zudem arbeitet die offenbarungsgemäße piezoelektrische Antriebseinheit sehr leise. Dies stellt einen wichtigen Nutzen für einen Anwender dar, insbesondere für Situationen mit mehrstündigen Infusionen und/oder bei mobil getragenen (Spritzen-) Pumpen. Ferner bietet die gesamthaft piezoelektrische Antriebsumsetzung gegenüber den herkömmlichen Bauweisen die weiteren produktseitigen, nicht zuletzt hinsichtlich mobil getragener (Spritzen-) Pumpen, sehr wertvollen Nutzen eines geringen Bauraumbedarfs sowie eines geringen Energieverbrauchs und einer geringen betriebsbedingten Wärmedissipation.

Vorzugsweise, alternativ oder kumulativ, kann der zumindest eine zweite Piezo-Aktor eingerichtet sein, durch dessen Ausdehnen die erste Klemmvorrichtung in den ersten Entkopplungszustand freizugeben. Insbesondere kann der zumindest eine zweite Piezo-Aktor eingerichtet sein, den ersten Entkopplungszustand zu bewirken und/oder durch dessen Zusammenziehen den ersten Kopplungszustand zu bewirken.

Vorzugsweise, alternativ oder kumulativ, kann der zumindest eine zweite Piezo-Aktor eingerichtet sein, durch dessen Ausdehnen mittels der ersten Klemmvorrichtung in einer Querrichtung freigebend und/oder klemmend auf die Schubstange zu wirken. Insbesondere kann der zumindest eine zweite Piezo-Aktor eingerichtet sein, eine in der ersten Klemmvorrichtung wirkende Druckkraft oder eine resultierende Rückstellkraft, welche einen in der Querrichtung auf die Schubstange wirkenden Vektoranteil aufweist, vorzusehen.

Vorzugsweise, alternativ oder kumulativ, kann die Steuereinheit eingerichtet sein, den ersten Kopplungszustand und nachfolgend den ersten Entkopplungszustand vorzusehen. Dabei ist der erste Kopplungszustand vorzusehen (bzw. vorgesehen), um einen Schlittenvorschub des Schlittens gemäß dem Längshub der von einer axialen Ausgangsposition bewegbaren Schubstange zu erzeugen, insbesondere einen Schlittenvorschub des Schlittens auf eine in Längsrichtung (inkrementale, d.h. sukzessive vorgeschobene) Vorschubposition des Schlittens zu erzeugen. Nachfolgend ist der erste Entkopplungszustand vorzusehen (bzw. vorgesehen), um die Schubstange für eine von dem Schlitten getrennte Bewegung freizugeben. Dabei kann insbesondere die getrennte Bewegung eine Rückzugsbewegung der Schubstange zurück auf deren Ausgangsposition bei gleichzeitigem Verbleib des Schlittens auf der (inkrementalen) Vorschubposition betreffen.

Vorzugsweise, alternativ oder kumulativ, kann die piezoelektrische Antriebseinheit weiterhin eine Führungsschiene und eine zweite Klemmvorrichtung des Schlittens aufweisen. Dabei ist die Führungsschiene parallel zu der Schubstange an dem Antriebsrahmen vorgesehen. Dabei ist die zweite Klemmvorrichtung des Schlittens mittels des zumindest einen zweiten Piezo-Aktors und/oder zumindest eines mit der Steuereinheit verbundenen dritten Piezo-Aktors aktuierbar. Ferner ist dabei die zweite Klemmvorrichtung eingerichtet, um zwischen einem zweiten Kopplungszustand und einem zweiten Entkopplungszustand zu wechseln. Dabei ist der zweite Kopplungszustand vorzusehen (bzw. vorgesehen), eine laufhemmende Sicherung des Schlittens an der Führungsschiene gegen nicht intendierte (bzw. unbeabsichtigte) Axialbewegungen zu bewirken. Dabei ist der zweite Entkopplungszustand vorzusehen (bzw. vorgesehen), eine lauffreigebende Entsicherung des Schlittens von der Führungsschiene zu bewirken. Dies dient in besonders vorteilhafter Weise einer Sicherung des Schlittens gegen ein Verrutschen in der verfahrbaren Längsrichtung.

Insbesondere bevorzugt kann dabei die Führungsschiene in einer radialen Richtung (bzw. entlang einer bzw. in einem spitzen Winkel zu einer Querrichtung) mit einem sogenannten Strichschliff ausgebildet sein. Dies dient dazu, einen zusätzlichen Oberflächenwiderstand zur weiteren Sicherung gegen Verrutschen der geklemmten Oberflächen zueinander aufzubauen.

Die vorzugsweise zusätzlich zu der ersten Klemmvorrichtung vorgesehene zweite Klemmvorrichtung bildet als eine Verstärkung der Querklemmung des Schlitten, um diesen gegen weitere angreifende Belastungen bzw. Kräfte zu sichern. Das Klemmen erfolgt auf der rippenförmigen Führungsschiene als einem dazu im Antriebsrahmen vorgesehenen Profil, vorzugsweise Aluminiumprofil.

Somit besteht ein vorteilhafter Nutzen der zweiten Klemmvorrichtung besteht in einer zusätzlichen Sicherung des Schlitten gegen axiales Verrutschen. Wenn der Schlitten die Schubstange im ersten Entkopplungszustand loslässt, damit diese zurückgezogen werden kann, um sodann den nächsten Längshub zwecks des inkrementalen Schlittenvorschubs (wiederholter Zyklus) auszuführen, sichert die zweite Klemmvorrichtung, dass wenn Kräfte an dem Schlitten angreifen, dieser immer noch gegen Verrutschen gesichert ist. Dies ist beispielsweise vorteilhaft, um die vorliegend offenbarte piezoelektrische Antriebseinheit bzw. das Gesamtsystem der in einer (Spritzen-)Pumpe eingebauten Antriebseinheit gegen außerplanmäßige mechanische Belastungen abzusichern. Beispielsweise kann die zusätzliche Sicherung für das Szenario eines unbeabsichtigten Herunterfallens bzw. eines Sturzes eines Patienten mit einem mobilen Gesamtsystem sich als von besonderem Vorteil erweisen.

Vorzugsweise, alternativ oder kumulativ, können der zumindest eine erste Piezo-Aktor bzw. der (optionale) zumindest eine zweite bzw. der (optionale) zumindest eine dritte Piezo-Aktor mit unterschiedlichen technischen Spezifikationen (bzw. Baugröße, Type, usw.) ausgebildet bzw. ausgelegt sein. Insbesondere betreffen diese etwaig unterschiedlichen technischen Spezifikationen konstruktiv relevante Parameter wie eine piezoelektrisch aktuierte bzw. nominelle Piezo-Kraft und/oder einen piezoelektrisch aktuierten bzw. nominellen Piezohub und/oder Dimensionen (Länge, Höhe, Breite) und/oder Material, Beschichtung, Anzahl der Lagen und dergleichen. Dies dient einer besonders kompakten Bauweise und optimalen, variablen Auslegung der Funktion.

Vorzugsweise, alternativ oder kumulativ, kann dabei der (erste / zweite / dritte) Piezohub bzw. die (insb., aber nicht limitierend: unipolare) Auslenkung bzw. die Längsausdehnung, insbesondere der nominale bzw. theoretische bzw. maximale Wert derselben, ca. 5 bis 45 Mikrometer, weiter bevorzugt ca. 8 bis 16 Mikrometer, insbesondere ca. 9 Mikrometer betragen. Alternativ oder kumulativ weiter bevorzugt kann die (auf die Länge des Piezo-Aktors bezogene) Auslenkung bzw. die relative Längsausdehnung des (ersten / zweiten / dritten) Piezo-Aktors, insbesondere der nominale bzw. theoretische bzw. maximale Wert derselben, ca. 0,1 %, insbesondere ca. 0,2 % betragen.

Insbesondere kann der (erste / zweite / dritte) Piezo-Aktor eine Querschnittsfläche 5 x 5 mm²; eine Länge von 9 mm; eine elektrische Kapazität von 800 nF; und/oder eine Piezo-Kraft von 850 Newton aufweisen [bspw. Piezo-Aktor der technischen Type "PB 5.9" (Piezotechnics GmbH, Deutschland)]. Alternativ oder kumulativ bevorzugt kann der Piezo-Aktor zumindest teilweise metallgehäust und/oder mit einer, vorzugsweise polymeren oder keramischen, Schutzbeschichtung ausgebildet sein, was einen Schutz gegen eine hohe Luftfeuchte bietet.

Insbesondere kann der zumindest eine (erste / zweite / dritte) Piezo-Aktor eine jeweilige Vielzahl bzw. ein Vielfaches des (ersten / zweiten / dritten) Piezo-Aktor meinen. Dabei kann bspw. eine paarige Anordnung bevorzugt sein, d.h. bspw. zwei (baugleiche) erste / zweite / dritte Piezo-Aktoren nebeneinander oszillieren. In entsprechender Weise sind vorteilhaft die Piezo-Kräfte des ersten / zweiten / dritten Piezo-Aktors um das Vielfache erhöht bzw. verstärkt.

Insbesondere kann dabei die zweite Klemmvorrichtung eingerichtet sein, stromlos den zweiten Kopplungszustand vorzusehen. Mit anderen Worten, ist die zweite Klemmvorrichtung bzw. der Schlitten mittels dieser an der Führungsschiene gegen nicht intendierte Axialbewegungen laufhemmend gesichert. Dies sichert die piezoelektrische Antriebseinheit vorteilhaft gegen mechanische Beschädigung und/oder nicht intendierte(n) Betätigung (bzw. Betrieb) durch ein Verrutschen. Dies dient insb. dazu, eine unbeabsichtigte, ggf. prohibitive, Medikamentengabe an einen Patienten bspw. aufgrund eines unbeabsichtigten Falles der (Spritzen-)Pumpe im Vorfeld zu vermeiden, was insb. im Falle einer mobil für eine Langzeitinfusion getragenen (Spritzen-)Pumpe ein abzusicherndes Unfallrisiko betrifft.

Vorzugsweise, alternativ oder kumulativ, kann die Steuereinheit eingerichtet sein, den zweiten Entkopplungszustand jeweilig zeitlich vorausgehend oder gleichzeitig zu dem ersten Kopplungszustand vorzusehen, um einen Schlittenvorschub des Schlittens gemäß dem Längshub der von einer axialen Ausgangsposition bewegbaren Schubstange auszuführen.

Vorzugsweise, alternativ oder kumulativ, kann die Steuereinheit eingerichtet sein, den zweiten Kopplungszustand jeweilig zeitlich direkt nachfolgend oder gleichzeitig zu dem ausgeführten Schlittenvorschub vorzusehen.

Vorzugsweise, alternativ oder kumulativ, kann zumindest eine aus der Längshub-Vorrichtung und/oder der ersten Klemmvorrichtung und/oder der zweiten Klemmvorrichtung als eine jeweilige Festkörpergelenk-gelagerte Mechanik ausgebildet sein. Insbesondere kann dabei die jeweilige Festkörpergelenk-gelagerte Mechanik hinsichtlich der Oszillation zumindest eines (zugehörigen) Piezo-Aktors aus dem zumindest einen ersten, zweiten und/oder dritten Piezo-Aktor dauerfest ausgebildet sein. Dabei meint der Begriff der Festkörpergelenk-gelagerten Mechanik den Einsatz von Festkörpergelenken, d.h. elastisch verformbarer bzw. verformter spezieller Bereiche eines (Bau)Teils zur Bewegungslenkung bzw. im Sinne einer funktionalen Kinematik. Dieses Merkmal dient der weiteren Erhöhung der Genauigkeit und der Robustheit der Antriebseinheit als einem System bzw. eine Anordnung. Insofern zeichnet sich die Festkörpergelenk-gelagerte Mechanik durch seine spielfreie Eigenschaft bzw. Bauart aus. Gegenüber dem einleitend dargestellten Stand der Technik mit einer insb. auf Schiebern, Kugeln, Federn, usw. basierenden spielbehafteten Bauart fällt bei der vorliegend weiter bevorzugten spielfreien Bauart mit Festkörpergelenken die Abstimmung der Toleranzen weitaus leichter. Das macht die Konstruktion bzw. Bauart günstiger. Zudem zeichnet sich die bevorzugte Ausführungsform durch einen weitaus kompakteren Bauraum und weniger Teile aus. Insbesondere dient die weiter bevorzugte dauerfeste (bzw. dauerelastische) Ausbildung bzw. Auslegung der jeweiligen Festkörpergelenk-gelagerten Mechanik der Verschleiß- und Wartungsfreiheit bzw. der Haltbarkeit ohne Erscheinungen von Materialermüdung. In eine entsprechende Auslegung im Rahmen zulässiger Spannungen im dauerfesten Bereich (gemäß Wöhler-Kurven) können eine mechanische Last, eine Materialwahl, eine spezifische Geometrie sowie eine erfahrbare (bzw. simulierbare) Verformung des jeweiligen Festkörpergelenks einfließen. Durch die elastische Verformung des Festkörpergelenkes der Festkörpergelenk-gelagerten Mechanik ergibt sich, wie bei einer Blattfeder, eine rückstellende Kraft. Daraus ergibt sich der Vorteil, dass man auf anderweitige rückstellende Vorspannungselemente, beispielsweise eine Feder, und auf die für diese benötigten Lagerungselemente, beispielsweise eine Buchse, verzichten kann.

Vorzugsweise, alternativ oder kumulativ, kann in dem Falle, dass die erste Klemmvorrichtung als eine Festkörpergelenk-gelagerte Mechanik ausgebildet ist, die erste Klemmvorrichtung eine Biegefeder aufweisen. Dabei ist die Biegefeder in einem Schubstangen-Klemmabschnitt zwischen der Schubstange und dem Schlitten angeordnet. Ferner ist dabei die Biegefeder an dem Antriebsrahmen in einem Biegefeder-Drehpunkt drehbar gelagert ist, um einen hin zu dem Schubstangen-Klemmabschnitt verlaufenden Biegefeder-Hebelarm auszubilden. Vorzugsweise kann dabei die Biegefeder weiterhin an dem Schlitten in einem Anlagepunkt in Verlängerung des Biegefeder-Hebelarms rückstellend gegengelagert sein. Ferner ist dabei die Biegefeder ausgebildet, um sich bei piezoelektrisch aktuiertem Ausdehnen des zweiten Piezo-Aktors in der Querrichtung derart zu verformen, dass die Schubstange von dem Schlitten in den ersten Entkopplungszustand freigegeben wird. Weiter bevorzugt kann dabei die Biegefeder ausgebildet sein, um bei deren Verformung eine Vorspannung mit einer in der Querrichtung resultierenden Biegefeder-Rückstellkraft (derart) zu bewirken, dass bei Zusammenziehen des zweiten Piezo-Aktors der Schlitten zurück an die Schubstange in den ersten Kopplungszustand koppelt. Der Haltemechanismus der ersten Klemmvorrichtung funktioniert, indem die (zunächst) vorgeschobenen Schubstange (sodann) festgehalten wird, während sich das Festkörpergelenk in seine ursprüngliche Position zurückstellt.

Vorzugsweise, alternativ oder kumulativ, kann in dem Falle, dass die zweite Klemmvorrichtung als eine Festkörpergelenk-gelagerte Mechanik ausgebildet ist, diese einen schwenkarmartigen Klemmhebel aufweisen. Dabei ist der Klemmhebel um die Längsachse der Linearführung drehbar gelagert, um bei piezoelektrisch aktuiertem Ausdehnen des zumindest einen zweiten Piezo-Aktors und/oder dritten Piezo-Aktors durch dessen Auslenkungskraft den zweiten Kopplungzustand zu bewirken. Weiter bevorzugt kann dabei der Klemmhebel ausgebildet sein, um eine an dem Klemmhebel angreifende Auslenkungskraft des zumindest einen zweiten Piezo-Aktors und/oder dritten Piezo-Aktors in eine, dazu Hebelgesetz-mäßig verstärkte, an der Führungsschiene ansetzende Führungsschienen-Klemmkraft zu übersetzen. Vorzugsweise kann dabei ein Klemmhebel um einen Klemmhebel-Drehpunkt vorgesehen sein, welcher mit einem Zentrum der Linearführung zusammenfällt. Insbesondere wenn sich der zumindest eine dritte Piezo-Aktor in Querrichtung ausdehnt, bewirkt dies eine Verdrehung des Klemmhebels um den Drehpunkt mit einer entsprechenden Klemmhebel-Hebellänge. Dabei entspricht die Klemmhebel-Hebellänge der Strecke von dem Klemmhebel-Drehpunkt bis zu einem Druckpunkt (bei Expandieren) des zumindest einen dritten Piezo-Aktors auf den Klemmhebel.

Vorzugsweise, alternativ oder kumulativ, kann in dem Falle, dass die Längshub-Vorrichtung als eine Festkörpergelenk-gelagerte Mechanik ausgebildet ist, die Längshub-Vorrichtung ein zweiseitiges Hebelwippenelement aufweisen. Dabei ist das Hebelwippenelement in dessen zentralen Hebelwippen-Mittenabschnitt an einem hinsichtlich einer Richtung des Schlittenvorschubs rückwärtigen Ende des Antriebsrahmen, insbesondere an einer hinteren Rahmenplatte, um eine zur Längsrichtung orthogonale Hebelwippen-Drehachse bzw. an einem Hebelwippen-Drehpunkt schwenkbar gelagert. Dabei kann vorzugsweise der feste Hebelwippen-Drehpunkt in Form einer Verschraubung des Festkörpergelenks an dem Antriebsrahmen der Antriebseinheit vorgesehen sein. Bei dem Festkörpergelenk, insb. hier dem Hebelwippenelement, handelt es sich um ein Element aus einem elastischen Material, insbesondere Metall, welches immer nur elastisch ausgelenkt bzw. verformt wird. Ferner bildet dabei das Hebelwippenelement dazu zweiseitig an den gegenüberliegenden Enden jeweilig einen kurzen Hebelwippenarm und einen langen Hebelwippenarm aus, um bei piezoelektrisch aktuiertem Ausdehnen des zumindest einen ersten Piezo-Aktors um einen ersten Piezohub den Längshub der Schubstange zu bewirken.

Weiter bevorzugt kann dabei das Hebelwippenelement ausgebildet sein, um den an dem kurzen Hebelwippenarm ansetzenden ersten Piezohub des ersten Piezo-Aktors in den, dazu Hebelgesetz-mäßig vergrößerten, Längshub der an dem langen Hebelwippenarm angeordneten Schubstange zu übersetzen.

Ein kurzer Hebelwippenarm wird als eine Strecke von dem Hebelwippen-Drehpunkt bis zu einem ersten Piezo-Aktor ausgebildet. Ein langer Hebelwippenarm wird als eine Strecke von dem Hebelwippen-Drehpunkt bis zur Schubstange ausgebildet. Um den Hebelwippen-Drehpunkt wird ein Hebel mit einer kurzen Hebellänge bzw. dem kurzen Hebelwippenarm in einer parallelen Richtung zu der Längsrichtung der Schubstange durch den ersten Piezohub (bspw. ca. 0,01 mm) um den oszillierenden, ersten Piezo-Aktor erzeugt. Die kurze Hebellänge übersetzt sich um den Hebelwippen-Drehpunkt mit der langen Hebellänge bzw. dem langen Hebelwippenarm zur Schubstange um. Vorzugsweise kann die kurze Hebellänge 4 mm bis 10 mm betragen, insbesondere ca. 7 mm betragen. Vorzugsweise kann die lange Hebellänge 10 mm bis 25 mm, weiter bevorzugt 11 mm bis 15 mm, insbesondere ca. 12 mm betragen. Ein Hebeleffekt des Festkörpergelenks wird entsprechend einem, als Quotient der langen Hebellänge zu der kurzen Hebellänge definierten, Übersetzungsverhältnis bewirkt. Vorzugsweise kann das Übersetzungsverhältnis als besagter Quotient in einem Bereich von 1,4 bis 4, weiter bevorzugt von 1,5 bis 2,0, insbesondere von 1,7 bis 1,8 liegen.

Gemäß besagtem Übersetzungsverhältnis resultiert eine entsprechende Reduktion einer von dem ersten Piezo-Aktor bewirkten Kraft bei gleichzeitiger entsprechender Erhöhung des in die Längsrichtung wirkenden Längshubs der Schubstange. Der Schubstangenhub führt jeweilig einen inkrementalen Schlittenvorschub in der Längsrichtung der Schubstange aus. Mit anderen Worten, lässt sich durch die Auslegung des Übersetzungsverhältnisses bzw. der kurzen und/oder der langen Hebellänge ein vorbestimmter Längshub der Schubstange und/oder eine vorbestimmte von dem ersten Piezo-Aktor bewirkte erste Piezo-Kraft darstellen. Vorzugsweise kann die erste Piezo-Kraft, welche axial auf die Schubstange als Förderkraft zum Vorschub wirkt, mindestens 50 Newton, weiter bevorzugt mindestens 100 Newton, insbesondere mindestens 200 Newton betragen.

Ein weiterhin genannter (bzw. zweitgenannter) Aspekt der vorliegenden Offenbarung betrifft ein Verfahren zum Erzeugen eines Schlittenvorschubs des Schlittens einer piezoelektrischen Antriebseinheit nach dem vorstehend erstgenannten Aspekt der vorliegenden Offenbarung. Zur Vermeidung von Wiederholungen wird hinsichtlich wesentlicher und bevorzugter technischer Merkmale aller weiterhin genannten Aspekte der vorliegenden Offenbarung auf die vorstehende Offenbarung zu dem erstgenannten Aspekt verwiesen. Dabei ist das offenbarungsgemäße Verfahren über die Steuereinheit der piezoelektrischen Antriebseinheit ausführbar und weist dabei nachfolgende Schritte auf, insbesondere in Weise einer wiederholbaren Schrittsequenz:
Ein anfänglicher Schritt betrifft eine Kopplung (bzw. ein Koppeln, Klemmen, temporäres Verbinden) des Schlittens an der Schubstange mittels der ersten Klemmvorrichtung des Schlittens in einen piezoelektrisch aktuierten ersten Kopplungszustand.

Sodann betrifft ein weiterer Schritt ein Erzeugen eines Schlittenvorschubs des Schlittens, insbesondere auf eine in Längsrichtung inkrementale Vorschubposition, gekoppelt mit einem Längshub der Schubstange von einer axialen Ausgangsposition bei mittels des zumindest einen ersten Piezo-Aktors piezoelektrisch aktuiertem Einwirken der Längshub-Vorrichtung. Insbesondere kann dabei der Schlittenvorschub einen auf eine in Längsrichtung inkrementale Vorschubposition betreffen.

Sodann betrifft ein weiterer Schritt eine Entkopplung (bzw. ein Entkoppeln, Lösen, temporäre Aufhebung der Klemmung) des Schlittens von der Schubstange mittels der ersten Klemmvorrichtung des Schlittens in einen piezoelektrisch aktuierten ersten Entkopplungszustand.

Sodann betrifft ein weiterer Schritt eine getrennte Bewegung der Schubstange bei mittels des zumindest einen ersten Piezo-Aktors piezoelektrisch aktuiertem Einwirken der Längshub-Vorrichtung. Insbesondere kann dabei die getrennte Bewegung eine Rückzugsbewegung der Schubstange von der in der Längsrichtung inkrementalen Vorschubposition zurück auf die Ausgangsposition betreffen.

Vorzugsweise, alternativ oder kumulativ, kann, insbesondere im Falle der vorzugsweise mit der zweiten Klemmvorrichtung des Schlittens vorgesehenen piezoelektrischen Antriebseinheit, das (noch über das Grundprinzip hinaus vorteilhaft weiterentwickelte) Verfahren zumindest einen der beiden weiteren über die Steuereinheit ausführbaren optionalen Schritte oder beide umfassen wie folgt:
Ein anfänglicher Schritt betrifft eine Kopplung (bzw. ein Koppeln, Klemmen, temporäres Verbinden) des Schlittens an der Schubstange mittels der ersten Klemmvorrichtung des Schlittens in einen piezoelektrisch aktuierten ersten Kopplungszustand.

Sodann betrifft ein optionaler, mittels der gegebenenfalls vorgesehenen zweiten Klemmvorrichtung des Schlittens ausführbarer Schritt, ein Wechseln aus einem zweiten Kopplungszustand zur laufhemmenden Sicherung des Schlittens an der gegebenenfalls vorgesehenen Führungsschiene in einen piezoelektrisch aktuierten zweiten Entkopplungszustand zur lauffreigebenden Entsicherung des Schlittens von der Führungsschiene.

Sodann betrifft ein weiterer Schritt ein Erzeugen eines Schlittenvorschubs des Schlittens, insbesondere auf eine in Längsrichtung inkrementale Vorschubposition, gekoppelt mit einem Längshub der Schubstange von einer axialen Ausgangsposition bei mittels des zumindest einen ersten Piezo-Aktors piezoelektrisch aktuiertem Einwirken der Längshub-Vorrichtung. Insbesondere kann dabei der Schlittenvorschub einen auf eine in Längsrichtung inkrementale Vorschubposition betreffen.

Sodann betrifft ein optionaler, mittels der gegebenenfalls vorgesehenen zweiten Klemmvorrichtung des Schlittens ausführbarer Schritt, ein Wechseln aus dem zweiten Entkopplungszustand zurück in den piezoelektrisch aktuierten zweiten Kopplungszustand zur laufhemmenden Sicherung des Schlittens an der gegebenenfalls vorgesehenen Führungsschiene, insbesondere auf der inkrementalen Vorschubposition. Hinsichtlich letztgenannten Schrittes ist anzumerken, dass mit dem Begriff des (Zurück-)Wechseln gemeint ist, dass im Falle der gegebenenfalls vorgesehenen zweiten Klemmvorrichtung des Schlittens sinnvollerweise beide zugehörigen optionalen Schritte ausgeführt werden (oder beide nicht ausgeführt werden, insofern die zweite Klemmvorrichtung bspw. interimsweise nicht verwendet werden könnte). Sprich, dass der eine erstgenannte optionale Schritt des Wechselns in den zweiten Entkopplungszustand (ausgehend von einem Zustand im zweiten Kopplungszustand) mit dem zweitgenannten optionalen Schritt des (Zurück-)Wechseln in den piezoelektrisch aktuierten zweiten Kopplungszustand einhergeht bzw. im funktionalen Zusammenhang steht, insbesondere wenn auf die wiederholbare Schrittsequenz bezogen.

Sodann betrifft ein weiterer Schritt eine Entkopplung (bzw. ein Entkoppeln, Lösen, temporäre Aufhebung der Klemmung) des Schlittens von der Schubstange mittels der ersten Klemmvorrichtung des Schlittens in einen piezoelektrisch aktuierten ersten Entkopplungszustand.

Sodann betrifft ein weiterer Schritt eine getrennte Bewegung der Schubstange bei mittels des zumindest einen ersten Piezo-Aktors piezoelektrisch aktuiertem Einwirken der Längshub-Vorrichtung. Insbesondere kann dabei die getrennte Bewegung eine Rückzugsbewegung der Schubstange von der in der Längsrichtung inkrementalen Vorschubposition zurück auf die Ausgangsposition betreffen.

Vorzugsweise, alternativ oder kumulativ, kann das Verfahren einen weiteren über die Steuereinheit ausführbaren Schritt umfassen wie folgt: Ein Steuern der Oszillation zumindest eines Piezo-Aktors aus dem zumindest einen, zweiten und/oder dritten Piezo-Aktor. Dabei kann insbesondere ein (Schritt des) Steuern(s) des Längshubs der an dem Antriebsrahmen mittels der Längshub-Vorrichtung oszillierend bewegbaren Schubstange erfolgen. Weiter bevorzugt kann Steuern stufenlos ausgebildet sein.

Ein weiterhin genannter Aspekt der vorliegenden Offenbarung betrifft eine medizintechnische (Spritzen-)Pumpe mit einer piezoelektrischen Antriebseinheit nach dem erstgenannten Aspekt der vorliegenden Offenbarung. Alternativ oder kumulativ kann die medizintechnische (Spritzen-)Pumpe ausgebildet und eingerichtet sein, das Verfahren zum Abgeben und/oder Dosieren medizinischer Flüssigkeiten nach dem vorstehend zweitgenannten Aspekt der vorliegenden Offenbarung auszuführen. Insbesondere kann dabei die die medizintechnische (Spritzen-)Pumpe als eine mobil von dem Anwender tragbare Infusionspumpe ausgeführt sein.

Ein weiterhin genannter Aspekt der vorliegenden Offenbarung betrifft eine erste Klemmvorrichtung und/oder die zweite Klemmvorrichtung für eine piezoelektrische Antriebseinheit nach dem erstgenannten Aspekt der vorliegenden Offenbarung. Insbesondere kann dabei die erste Klemmvorrichtung und/oder die zweite Klemmvorrichtung zur Verwendung als zugehöriges Bauteil (Zubehörteil bzw. Nachrüstteil bzw. Ersatzteil) ausgebildet sein. Alternativ oder kumulativ kann dabei die erste Klemmvorrichtung und/oder die zweite Klemmvorrichtung zur Verwendung in dem Verfahren zum Abgeben und/oder Dosieren medizinischer Flüssigkeiten nach dem vorstehend zweitgenannten Aspekt der vorliegenden Offenbarung ausgebildet sein.

Ein weiterhin genannter Aspekt der vorliegenden Offenbarung betrifft ein Computerprogramm auf einem Datenträger (bzw. maschinenlesbaren Speichermedium), eingerichtet zur Durchführung des offenbarungsgemäßen Verfahrens zum Abgeben und/oder Dosieren medizinischer Flüssigkeiten, wobei das Verfahren die über die Steuereinheit ausführbaren Schritte, insbesondere die wiederholbare Schrittsequenz, umfasst bzw. ausführt.

Zusammenfassend stellt der Einsatz einer gesamthaft mittels Piezo-Aktoren (als solche kräftig, dauerhaft präzise, verschleißfrei) realisierten Antriebseinheit, d.h. insbesondere als Ersatz bzw. unter Verzicht des üblicherweise in der Antriebseinheit verwendeten motorgetriebenen Spindel-, Schreit- und/oder Schwingantriebs, in einer Spritzenpumpe einen wesentlichen Gedanken der vorliegenden Offenbarung dar. Eine (piezoelektrisch) axial-oszillierende (bzw. in Längsrichtung oszillierte) Schubstange (deren Längshub durch den zumindest einen ersten Piezo-Aktor angeregt) schiebt oder zieht einen Schlitten in die gewünschte (Vorschub-) Richtung. Dazu werden die Schubstange (bzw. die Längshub-Vorrichtung) sowie die erste Klemmvorrichtung bzw. vorzugsweise ferner die zweite Klemmvorrichtung jeweils mittels des zugehörigen zumindest einen (ersten / zweiten / dritten) Piezo-Aktors bewegt.

Vorzugsweise, alternativ oder kumulativ, kann diese piezoelektrische Aktuierung (bzw. Betätigung, Auslösung, piezoelektrisch aktuierte Oszillation), abhängig von der Amplitudenhöhe der Oszillation, in stufenlos einstellbarer Genauigkeit (bis zum nominellen maximalen Piezohub des zugehörigen, jeweilig verwendeten (ersten / zweiten / dritten) Piezo-Aktors ausgeführt werden. Demzufolge ist von besonderem Vorteil, dass insbesondere die Genauigkeit des Antriebes (der kleinstmögliche Längshub der Schubstange bzw. Schlittenvorschub) stufenlos über die (piezoelektrisch aktuierte) Verformung der ersten Piezo-Aktors einstellbar sein kann.

In einer besonders bevorzugten Ausführungsform bewegen die unterschiedlichen (der zumindest eine erste bzw. zweite bzw. dritte) Piezo-Aktore(n), sozusagen in einem funktionalen bzw. mechanischen Zusammenspiel, mittels unterschiedlicher (der ersten und der zweiten) Klemmvorrichtungen zur abwechselnden Sicherung des Schlittens zum einen an der axial-oszillierten Schubstange und zum anderen an der Führungsschiene als einem über den Antriebsrahmen feststehenden Profil. Bei passender Umschaltung über die Steuereinheit wird der Schlitten dann um den gewünschten Schlittenvorschub in Längsrichtung bewegt bzw. (schrittweise, taktweise, zyklusweise) verfahren. Mit anderen Worten, wird durch mittels der Steuereinheit bewirktem ,Umgreifen' in Art eines koordinierten zeitweisen (bzw. phasenweisen) Verklemmens bzw. Koppelns des Schlittens an der Schubstange und/oder an der Führungsschiene ein (inkrementaler) Schlittenvorschub in die gewünschte Längsrichtung erzeugt bzw. resultiert eine (Antriebs-)Bewegung.

Vorzugsweise, alternativ oder kumulativ, können jeweilig die Längshub-Vorrichtung) und/oder die erste Klemmvorrichtung und/oder die zweite Klemmvorrichtung als die Festkörpergelenk-gelagerte (Hebel-)Mechanik(en) ausgebildet sein. Dies erlaubt in besonders vorteilhafter Weise, die im Mikrometerbereich kleinen Piezo-Hübe des jeweiligen zugehörigen (ersten / zweiten / dritten) Piezo-Aktors (sogar im Dauerbetrieb) spielfrei in größere (Nutz-) Hübe umzusetzen. Weiter ist als besonders vorteilhaft zu sehen, dass mittels der bevorzugten Festkörpergelenk-gelagerten (Hebel-)Mechanik(en) entsprechend der sehr hohen Anforderungen an die Betriebssicherheit solcher medizintechnischen Antriebseinheiten ausreichende Haltekräfte hinsichtlich der verfahrbaren Bauteile bzw. des Schlittens generiert werden.

Zusätzlich kann die offenbarungsgemäß vorgeschlagene Antriebseinheit auf einfache Weise als passiv sicher, d.h. stromlos gesperrt, ausgelegt werden, um eine medizintechnische (Spritzen-)Pumpe im Vorfeld gegen eine ungewollte Medikamentenabgabe effektiv abzusichern.

Allgemein ergeben sich, wie vorstehend bereits dargelegt, vielfältigste technische Vorteile, die sich auf die festkörperphysikalisch bedingte, dauerhafte Feinstpräzision der piezoelektrisch hervorgerufenen Oszillation bzw. Expansion, selbst bei winzigsten Bewegungsamplituden bzw. Piezohüben des (ersten / zweiten / dritten) Piezo-Aktors, zurückführen lassen. Die geringe Größe des Piezo-Aktors trägt auch dazu bei, die Gesamtgröße des Antriebseinheit und demgemäß der daraus resultierenden (Spritzen-) Pumpe zu verringern. Hinsichtlich einer Spritzenpumpe können ein langsamer Vorschub bzw. ein Verfahren des Kolbenstößels (wenige Millimeter pro Stunde) bevorzugt sein. Dabei kann in einem stabil, sauberen, ruhigen Betrieb bspw. eine Förderrate von minimal 0,01 ml pro Stunde vorgesehen sein. Aufgrund der bereits dargelegten offenbarungsgemäßen Energieeffizienz ist eine längere bis lange, bspw. mehrere Tage überspannende, Tragezeit im Falle einer mobilen (d.h. von einem Patienten mitgeführten / getragenen) Pumpe zu dessen Konvenienz realisierbar.

Der Fachmann versteht dabei, dass auf Grundlage der vorliegenden Offenbarung andere Einsatzgebiete als für eine medizintechnische Infusionspumpe denkbar sind. Während ein Schwerpunkt der denkbaren Anwendungsbereiche der vorliegenden Offenbarung in der präzisen Dosierung einer, in der Applikationsform einer Spritze vorkonfektioniert vorgelegten, Infusionslösung eines bereits verdünnten zumindest einen Wirkstoffs bzw. Medikaments über einen längeren Zeitraum liegt, liegen für den Fachmann weitere Einsatzmöglichkeiten bzw. Verwendungen der vorliegenden Offenbarung nahe. So betrifft dies vielfältige Aufgabenstellungen der automatischen Feinstdosierung von fließfähigen Flüssigkeiten in der Produktion, beispielsweise in der Parfummischung, Herstellung von Pharmazeutika, usw. Insbesondere ist es denkbar, bei entsprechender Anpassung der Dimensionierung bzw. der technischen Parameter der vorliegend offenbarten Antriebseinheit, eine präzise Dosierung eines konzentriert(er) vorliegenden Wirkstoffs bzw. Medikaments in eine Lösung, insbesondere in eine stabilisierende Formulierung und/oder in eine Infusionslösung, in verdünnender Weise vorzunehmen. Beispielsweise kann dies in einer Operationssituation hinsichtlich einer grundsätzlich bzw. akut vorzunehmenden Anästhesie(maßnahme) und/oder einer lebenserhaltenden Stabilisierung eines Patienten nützlich sein. Insbesondere kann die Antriebseinheit auch dahingehend optimiert werden, eine punktuelle und/oder spontane Feinstdosierung in spezifisch gewünschten bzw. piezoelektrisch steuerbaren Dosierungsvolumina vorzunehmen. Darüberhinaus ist es denkbar, die präzise Antriebseinheit, insbesondere unter Umkehr der oben dargelegten Vorschubrichtung in Längsrichtung der Schubstange, zur gezielten automatischen Probennahme bzw. Blutentnahme medizintechnisch einzusetzen. Insbesondere liegen also Abwandlungen für den Einsatz als präzise, verschleißfreie Antriebseinheit geringster Baugröße für Dosierpumpen bzw. Applikationspumpen der (automatisierten) (Flüssig-) Mischung, Analytik, Herstellung, Montage, nahe.

### Kurzbeschreibung der Figuren

Die Offenbarung wird nachstehend anhand bevorzugter Ausführungsbeispiele unter Bezugnahme auf die beigefügten Zeichnungen näher beschrieben. Es zeigen:
Fig. 1 eine perspektivische Ansicht einer piezoelektrischen Antriebseinheit für eine (ihrerseits nicht dargestellte) medizintechnische Spritzenpumpe gemäß einem ersten bevorzugten Ausführungsbeispiel nach der vorliegenden Offenbarung;
Fig. 2 eine weitere (zu der Ansicht der Fig. 1 im Raum gedrehte) perspektivische Ansicht der piezoelektrischen Antriebseinheit gemäß dem ersten Ausführungsbeispiel;
Fig. 3a bzw. 3b zwei jeweilige Aufsichten der piezoelektrischen Antriebseinheit gemäß dem ersten Ausführungsbeispiel, einen bei Verwendung in der zugehörigen medizintechnischen Spritzenpumpe relevanten Kolbenstößel-Verfahrweg der Antriebseinheit veranschaulichend;
Fig. 4b bis 4d (unter Bezugnahme auf die in der Aufsicht der Fig. 4a angezeichnete Ansichtsschnittlinie B-B) unterschiedliche Darstellungen einer Längshub-Vorrichtung der piezoelektrischen Antriebseinheit gemäß dem ersten Ausführungsbeispiel, wie hier beispielhaft als eine bevorzugte Festkörpergelenk-gelagerte Mechanik ausgebildet:

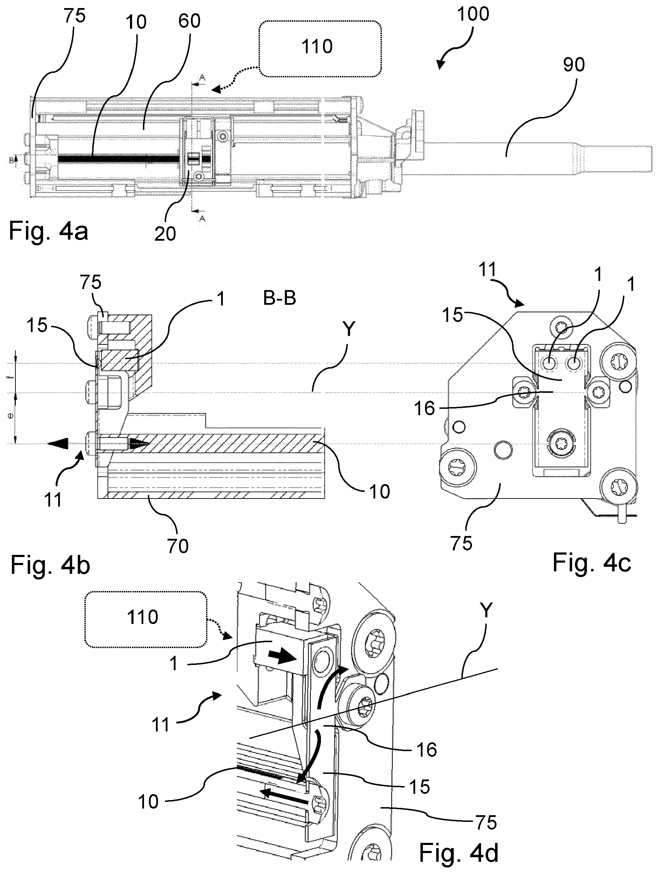

   Fig. 4b eine Querschnittsansicht in Schnittebene B-B;
   Fig. 4c eine zugehörige Rückansicht; und
   Fig. 4d eine perspektivische Aufsicht auf eine andere Schnittebene zur Veranschaulichung der kinematischen Funktionsweise der, mittels eines ersten Piezo-Aktors piezoelektrisch aktuierten, Längshub-Vorrichtung;

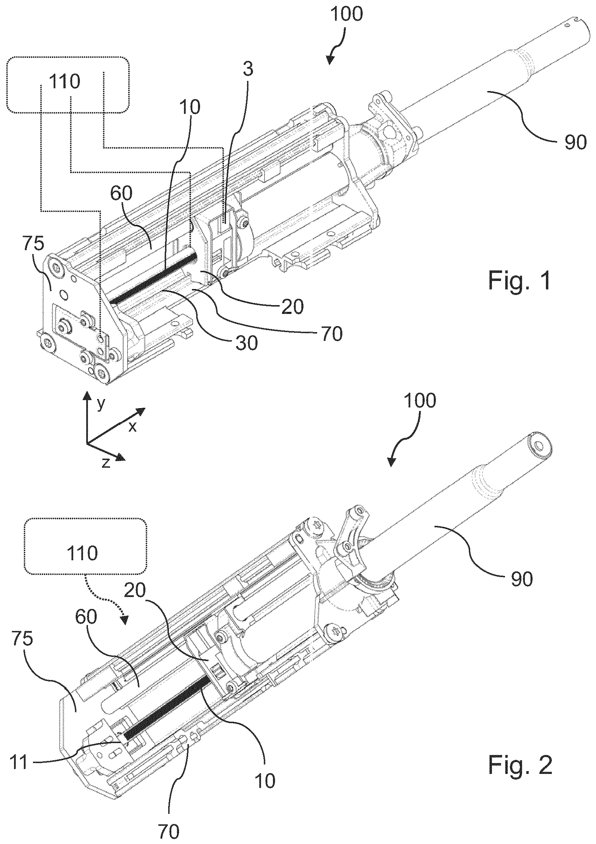

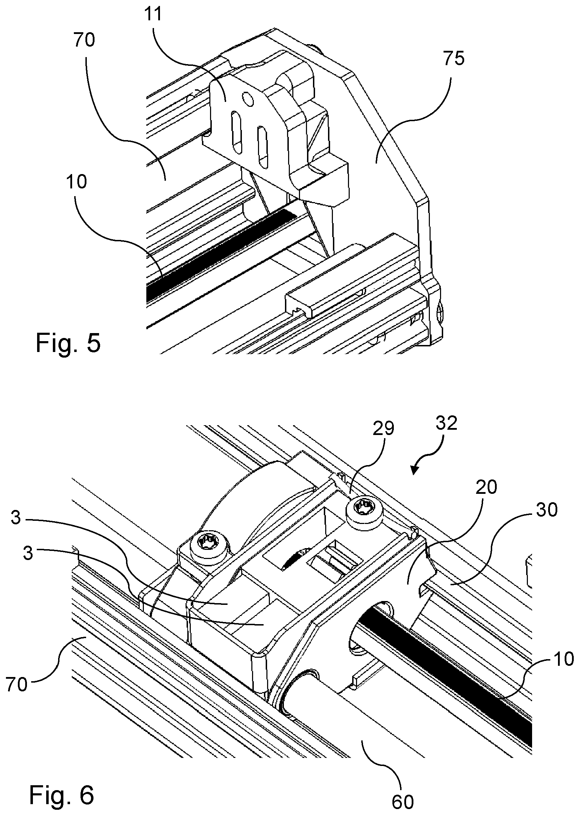

Fig. 5 ein vergrößerter Ausschnitt der perspektivischen Ansicht der Fig. 2 (dazu im Raum gedreht) der piezoelektrischen Antriebseinheit gemäß dem ersten Ausführungsbeispiel, auf den Bereich der Längshub-Vorrichtung für eine Schubstange fokussierend;
Fig. 6 ein vergrößerter Ausschnitt der perspektivischen Ansicht der Fig. 1 (dazu im Raum gedreht) der piezoelektrischen Antriebseinheit gemäß dem ersten Ausführungsbeispiel, fokussierend auf den Bereich einer zweiten Klemmvorrichtung für einen auf der Schubstange verfahrbaren Schlitten, eingerichtet zu dessen laufhemmender Sicherung an einer Führungsschiene;

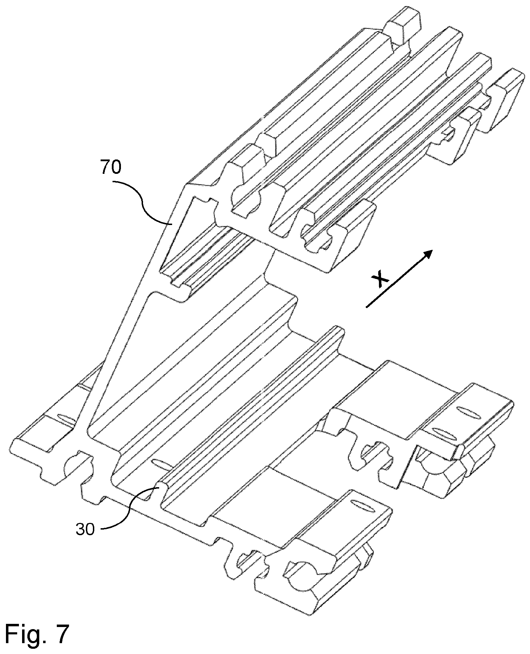

Fig. 7 eine perspektivische Ansicht eines (in Art einer Explosionszeichnung isoliert sowie in einem profilartigen Querschnitt dargestellten) Antriebsrahmens der piezoelektrischen Antriebseinheit gemäß dem ersten Ausführungsbeispiel, insbesondere die Führungsschiene zeigend;

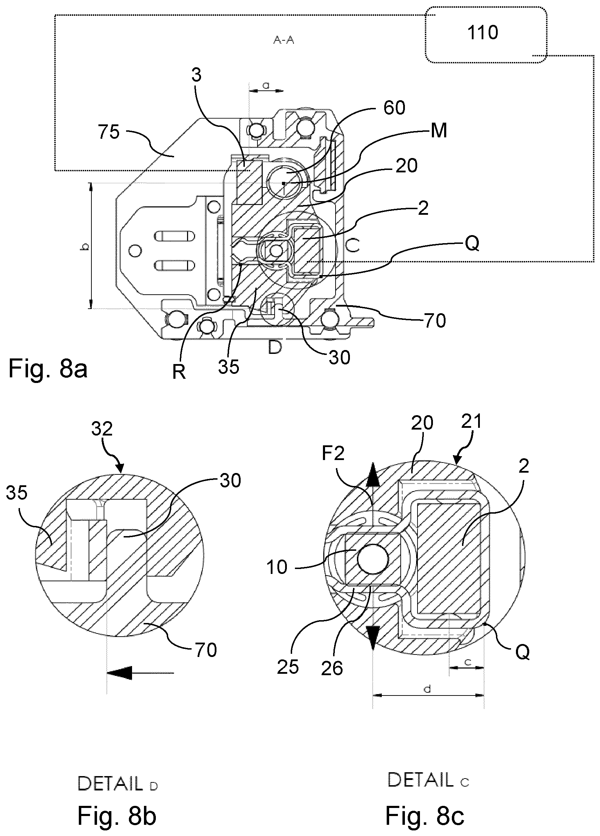

Fig. 8a bis 8c (unter Bezugnahme auf die in der Aufsicht der Fig. 4a angezeichnete Ansichtsschnittlinie A-A) unterschiedliche Darstellungen einer ersten Klemmvorrichtung sowie einer zweiten Klemmvorrichtung des Schlittens der piezoelektrischen Antriebseinheit gemäß dem ersten Ausführungsbeispiel, wie hier beispielhaft als weitere zwei bevorzugte Festkörpergelenk-gelagerte Mechaniken ausgebildet:
   Fig. 8a eine Querschnittsansicht in Schnittebene A-A (s. Fig. 4a), die erste Klemmvorrichtung sowie die zweite Klemmvorrichtung zeigend;
   Fig. 8b ein zugehöriger Detailausschnitt C aus der Fig. 8a hinsichlich der zweiten Klemmvorrichtung; und
   Fig. 8c ein zugehöriger Detailausschnitt D aus der Fig. 8a hinsichtlich der dritten Klemmvorrichtung;

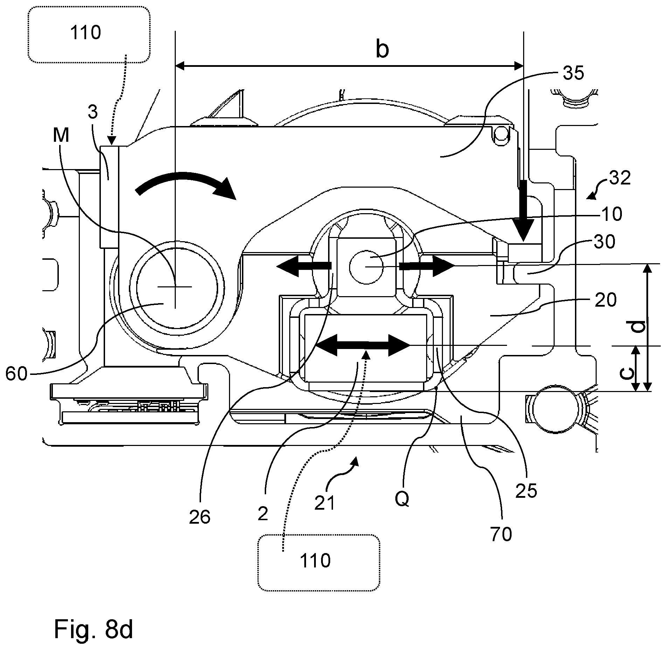

Fig. 8d eine andere (im Wesentlichen zu Fig. 8a korrespondierende) Querschnittsansicht zur Veranschaulichung der kinematischen Funktionsweisen der, mittels eines zweiten Piezo-Aktors piezoelektrisch aktuierten, ersten Klemmvorrichtung bzw. der, mittels eines dritten Piezo-Aktors piezoelektrisch aktuierten, zweiten Klemmvorrichtung der piezoelektrischen Antriebseinheit gemäß dem ersten Ausführungsbeispiel;

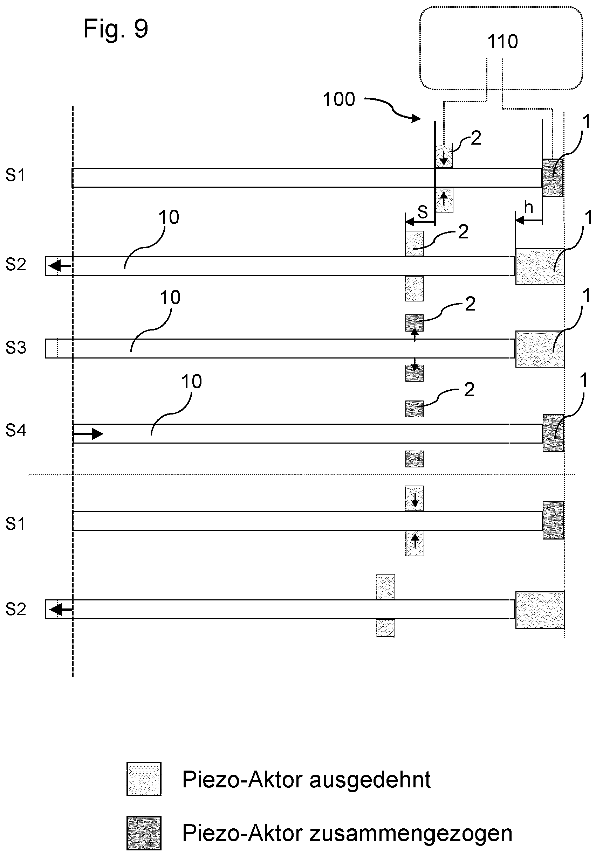

Fig. 9 ein zweites Ausführungsbeispiel der offenbarungsgemäßen piezoelektrischen Antriebseinheit, zur schematischen Veranschaulichung der zeitlichen Abfolge bzw. Koordination von mittels einer Steuereinheit ausführbaren (Arbeits-) Schritten im Grundprinzip, d.h. bei Berücksichtigung nur einer ersten Klemmvorrichtung; bzw. zur schematischen Veranschaulichung eines zugehörigen Verfahrens (betreffs wesentlicher Schritte eines Zyklus) zum Abgeben und/oder Dosieren medizinischer Flüssigkeiten;

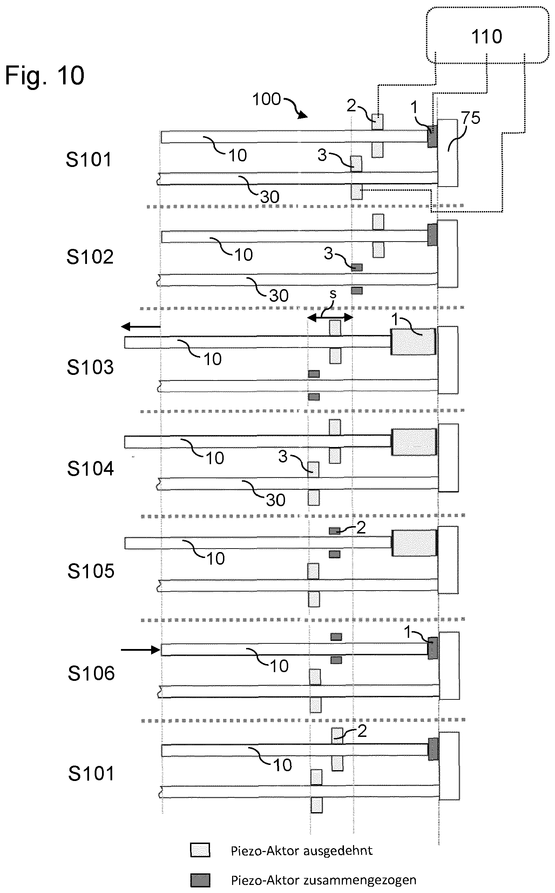

Fig. 10 ein drittes Ausführungsbeispiel der offenbarungsgemäßen piezoelektrischen Antriebseinheit zur schematischen Veranschaulichung der zeitlichen Abfolge bzw. Koordination von mittels einer Steuereinheit ausführbaren (Arbeits-) Schritten, den Fall einer zu dem ersten Ausführungsbeispiel (vgl. Figuren 1 bis 8d) identischen oder vergleichbaren Antriebseinheit mit einer bevorzugten zweiten Klemmvorrichtung veranschaulichend; bzw. zur schematischen Veranschaulichung des zugehörigen Verfahrens (inklusive optionaler, d.h. vorzugsweise ausgeführbarer,Schritte eines Zyklus); wobei ansonsten vergleichbare Bedingungen gegenüber dem zweiten Ausführungsbeispiel in Fig. 9 bestehen.

Die Figuren sind lediglich schematischer Natur und dienen ausschließlich dem Verständnis der Offenbarung. Die gleichen Elemente sind mit denselben Bezugszeichen bezeichnet.

### Beschreibung bevorzugter Ausführungsbeispiele

Nachstehend werden Ausführungsbeispiele der vorliegenden Offenbarung auf der Basis der zugehörigen Figuren beschrieben.

Figuren 1 bis 8d betreffen eine piezoelektrische Antriebseinheit 100 für eine medizintechnische Spritzenpumpe (nicht dargestellt; bspw. eine unter dem Markennamen "Space Plus Perfusor" vertriebene Infusionspumpe der vorliegenden Anmelderin B. Braun) gemäß einem ersten bevorzugten Ausführungsbeispiel nach der vorliegenden Offenbarung. Wiederum Figuren 9 und 10 veranschaulichen schematisch (im Grundprinzip, d.h. mit dem patentrechtlichen Charakter einer allgemeinen Offenbarung) die zeitliche Abfolge bzw. Koordination von mittels einer Steuereinheit 110 ausführbaren (Arbeits-) Schritten. Insbesondere veranschaulicht das erweiterte Schema der Fig. 10 ein dem ersten bevorzugten Ausführungsbeispiel zugehöriges Verfahren.

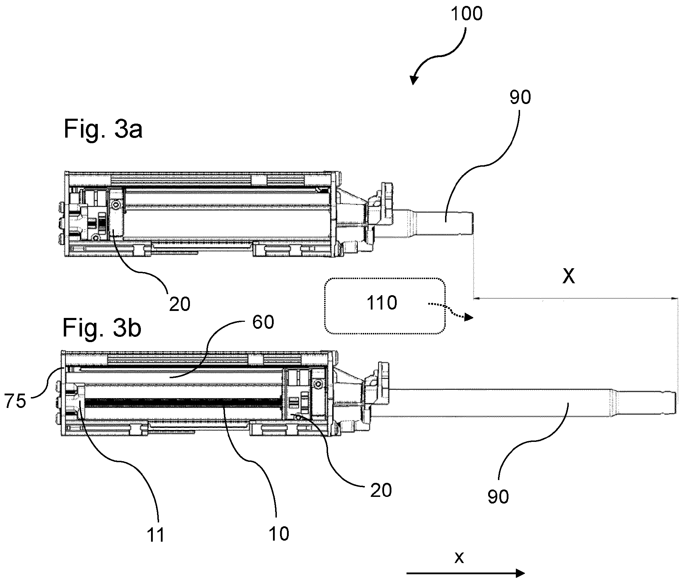

Wie den Gesamtansichten einer piezoelektrischen Antriebseinheit 100 in den Figuren 1 bis 4d zu entnehmen, ist diese mit einem Kolbenstößel 90 zum axialen (Längs-) Antrieb einer (nicht abgebildeten) medizintechnischen Spritzenpumpe oder vergleichbaren Pumpe zum Abgeben und/oder Dosieren medizinischer Flüssigkeiten ausgebildet. Dabei ist der Kolbenstößel 90 in der (axialen) Längsrichtung x um einen (gesamten bzw. kumulativen) Kolbenstößel-Verfahrweg X (siehe insb. Figuren 3a und 3b) verfahrbar. Dazu wird der Kolbenstößel 90 in einer Vielzahl von inkrementalen Vorschüben (vgl. unter Bezugnahme auf die Figuren 9 und 10: von Schlittenvorschüben s) schrittweise (bzw. taktweise, zyklusweise) verfahren.

Die piezoelektrische Antriebseinheit 100 (Figuren 1 bis 8d) hat weiter einen Antriebsrahmen 70 mit einer (hinteren) Rahmenplatte 75 an einem dem Kolbenstößel 90 gegenüberliegenden Ende. Der Antriebsrahmen 70 fungiert im Sinne eines feststehenden Profils oder eines stabilen äußeren Grundgehäuses. Die piezoelektrische Antriebseinheit 100 weist weiter eine Linearführung 60 auf. Dabei ist die in Art einer Führungswelle ausgeführte Linearführung 60 in dem Antriebsrahmen 70 in der Längsrichtung x verspannt.

Somit kann entlang der Linearführung 60 ein (an dieser) beweglich angeordneter Schlitten 20 verfahren werden, um die eigentliche Vorschubsfunktion zur Förderung abzubilden. Zusammen mit dem Schlitten 20 wird eine erste Klemmvorrichtung 21 des Schlittens gleichermaßen verfahren.

Die piezoelektrische Antriebseinheit 100 weist weiter eine Schubstange 10 auf. Die Schubstange 10 ist endseitig an dem Antriebsrahmen 70 bzw. an der Rahmenplatte 75 über eine Längshub-Vorrichtung 11 angeordnet. Dabei verlaufen die Linearführung 60 und die Schubstange 10 (im Wesentlichen, im Rahmen üblicher Lagetoleranzen) parallel zueinander.

Die piezoelektrische Antriebseinheit 100 weist weiter (zumindest) einen ersten Piezo-Aktor 1 der Längshub-Vorrichtung 11, einen zweiten Piezo-Aktor 2 der ersten Klemmvorrichtung 21 sowie einen dritten Piezo-Aktor 3 einer zweiten (optionalen) Klemmvorrichtung 32 auf. Und zwar besteht hier der erste Piezo-Aktor 1 (inbs. Fig. 4c), der zweite Piezo-Aktor 2 und der dritte Piezo-Aktor 3 (insb. Fig. 6) jeweils paarweise aus zwei solchen nebeneinander, d.h. in Weise eines ersten / zweiten / dritten Doppel-Piezo-Aktors.

Die jeweiligen ersten / zweiten / dritten Piezo-Aktoren 1, 2, 3 sind zum piezoelektrisch aktuierten Ausdehnen und Zusammenziehen um einen ersten / zweiten / dritten Piezohub mit einer (als gestrichelt umrandetes Symbolkästchen angedeuteten bzw. eingezeichneten) Steuereinheit 110 verbunden. Dazu erfolgt die Ansteuerung von der Steuereinheit 110 in den jeweiligen Figuren via der als gestrichelte Linien bzw. Pfeile dargestellten Steuerverbindungen (vgl. Figuren 1, 1, 3a/b, 4a/d, 8a/d; 9 und 10). Dabei ist vorzugsweise der erste / zweite / dritte Piezohub jeweils stufenlos (in Abhängigkeit von einer jeweilig angelegten Steuerspannung) verstellbar.

Die Schubstange 10 ist mittels der Längshub-Vorrichtung 11 in der Längsrichtung x um einen Längshub h oszillierend bewegbar eingerichtet bzw. wird (längs-)oszilliert bzw. vor- und zurückbewegt. Dabei wirkt zumindest ein erster Piezo-Aktor 1 der Längshub-Vorrichtung 11 auf die Schubstange 10 ein. Somit wird der Schlitten 20 entlang der Linearführung 60 aufgrund der mittels der Längshub-Vorrichtung 11 umgesetzten ersten Piezo-Kraft bewegt, indem der Schlitten 20 von der Schubstange 10 gedrückt (bzw. zurückgezogen wird).

Wie insb. den Figuren 4b bis 4d (unter Bezugnahme auf die in der Aufsicht der Fig. 4a angezeichnete Ansichtsschnittlinie B-B) entnehmbar, ist die Längshub-Vorrichtung 11 mit einem zweiseitigen Hebelwippenelement 15 (als eine bevorzugte Festkörpergelenk-gelagerte Mechanik) ausgebildet. Das Hebelwippenelement 15 ist in in dessen zentralen Hebelwippen-Mittenabschnitt 16 an der Rahmenplatte 75 um eine zur Längsrichtung x orthogonale Hebelwippen-Drehachse Y schwenkbar gelagert. An den gegenüberliegenden Enden des (zweiseitigen) Hebelwippenelements 15 sind jeweilig ein kurzer Hebelwippenarm f und einen langen Hebelwippenarm e ausgebildet.

Bei piezoelektrisch aktuiertem Ausdehnen des zumindest einen ersten Piezo-Aktors 1 um einen ersten Piezohub wird der Längshub h der Schubstange 10 zu bewirken (vgl. Figuren 9 und 10). Fig. 4d soll dabei anhand von eingetragenen Pfeilen die kinematische Funktionsweise bzw. die hebelmäßige Übersetzung darstellen. Bspw. kann eine für eine Spritzenpumpe bzw. übliche Infusionsspritzen benötigte maximale Förderkraft ca. 100 Newton betragen. Die Schubstange 10 ist ausgelegt, diese Förderkraft aufzubringen. Bspw. für den (zumindest einen) ersten Piezo-Aktor gewählte Piezo-Aktoren sind herstellerseitig zu maximal 850 Newton Piezo-Kraft und einem maximalen Piezohub von 0,009 mm spezifiziert. Mittels dem Hebelwippenelement 15 wird nun die Kraft (Piezo-Kraft umgesetzt auf Schubstangen-Kraft) reduziert und gleichzeitig der Hub (Piezohub umgesetzt auf Längshub als Schubstangen-Arbeitshub) vergrößert. Hier greifen dann gemäß dem Hebelgesetz die beiden Hebellängen des langen Hebelwippenarms e und des kurzen Hebelwippenarms f. In den Figuren 4a bis 4d ist eine beispielhafte Kraftuntersetzung von 1 : 2 dargestellt.

Die erste Klemmvorrichtung 21 des Schlittens 20 wird über den zweiten Piezo-Aktor 2 von der Steuereinheit 110 angesteuert. Nach erfolgter Klemmung kann der Schlitten 20 mit der (verfahrenden) Schubstange 10 in einem (temporären) ersten Kopplungszustand mitbewegt werden. Demzufolge (vgl. Figuren 9 und 10) erfolgt ein Schlittenvorschub des Schlittens gemäß dem Längshub der Schubstange. Damit die Schubstange 10 eine von dem Schlitten 20 unabhängige, separate Rückzugsbewegung zurück auf deren Ausgangsposition vollführen kann, ohne den Schlitten 20 wieder zurückzuziehen bzw. den erwünschten Schlittenvorschub unerwünscht rückgängig zu machen, wird die erste Klemmvorrichtung 21 wieder in einen ersten Entkopplungszustand versetzt.

Wie insb. den Figuren 8a (unter Bezugnahme auf die in der Aufsicht der Fig. 4a angezeichnete Ansichtsschnittlinie A-A), 8c, 8d entnehmbar, ist die erste Klemmvorrichtung 21 mit einer Biegefeder 25 (als eine bevorzugte Festkörpergelenk-gelagerte Mechanik) ausgebildet. Die Biegefeder 25 ist in einem Schubstangen-Klemmabschnitt 26 zwischen der Schubstange 10 und dem Schlitten 20 angeordnet. Ferner ist dabei die Biegefeder 25 an dem Antriebsrahmen 70 in einem Biegefeder-Drehpunkt Q drehbar gelagert, um einen hin zu dem Schubstangen-Klemmabschnitt 26 verlaufenden Biegefeder-Hebelarm d auszubilden. Die Biegefeder 25 ist weiterhin an dem Schlitten 20 in einem Anlagepunkt R in Verlängerung des Biegefeder-Hebelarms d rückstellend gegengelagert. Bei bzw. durch Ausdehnen des zweiten Piezo-Aktors 2 in der Querrichtung (siehe Fig. 1: y, z) wird die Biegefeder derart verformt, dass die Schubstange 10 von dem Schlitten 20 entkoppelt bzw. freigegeben wird. Die Verformung bewirkt dabei auch eine Vorspannung mit einer in der Querrichtung resultierenden Biegefeder-Rückstellkraft (Reactio zu F2, wie anhand der Pfeile angedeutet), so dass bei Zusammenziehen des zweiten Piezo-Aktors 2 der Schlitten 20 zurück an die Schubstange 10 in den ersten Kopplungszustand koppelt bzw. geklemmt wird.

Die piezoelektrische Antriebseinheit 100 gemäß dem bevorzugten ersten Ausführungsbeispiel weist weiterhin eine Führungsschiene 30 und eine zweite Klemmvorrichtung 32 des Schlittens 20 auf. Die Führungsschiene 30 ist in der Längsrichtung x bzw. parallel zu der Schubstange 10 an dem Antriebsrahmen 70 vorgesehen (insb. Fig. 7). Die zweite Klemmvorrichtung 32 des Schlittens 20, wie insb. den Figuren 6, 8a, 8b sowie 8d zu entnehmen, wird mittels des dritten Piezo-(Doppel-) Aktors 3 aktuiert, um zwischen einem zweiten Kopplungszustand und einem zweiten Entkopplungszustand zu wechseln. Für den temporären Zeitraum des (insb. aber auch stromlos eintretenden) zweiten Kopplungszustands ist der Schlitten 20 an der Führungsschiene 30 gegen nicht intendierte Axialbewegungen laufhemmend gesichert bzw. blockiert. Für den temporären Zeitraum des zweites Entkopplungszustands ist der Schlitten 20 von der Führungsschiene gelöst bzw. entsichert bzw. die zuvor bestehende Blockade zum freien Lauf in der Längsrichtung wieder aufgehoben.

Für eine Auslegung der zweiten Klemmvorrichtung 32 kann bevorzugt nicht die Förderkraft, sondern die möglich einwirkende Kraft beim Sturzfall angenommen werden. Insofern darf es in einem solchen Szenario nicht zum Losbrechen des Schlittens 20 kommen. Bspw. kann bei folgenden Annahmen eines solchen Szenarios: einer Fallhöhe von ca. 1 m, einem Gewicht einer Spritzenpumpe von ca. 2 kg sowie einer Aufpralldauer 10 ms; unter Anwendung der Kraftsstoß-Gleichung eine bei einem Aufprall einwirkende Kraft zu ca. 886 Newton abgeschätzt werden. Mindestens diese Klemmkraft, insbesondere größergleich 1.000 Newton, muss durch erste und/oder zweite Klemmvorrichtung(en) 21, 32 (alleine oder gemeinsam) aufgebracht werden.

Da zusätzlich noch ein Mindestwert für einen (zweiten und/oder dritten) Piezo-Hub (wegen Elastizitäten im jeweiligen Klemmmechanismus) realisiert werden muss, um ein sicheres Öffnen und Schließen zu gewährleisten, werden hier jeweils zwei (zweite bzw. dritte) Piezo-Aktoren eingesetzt und deren Piezo-Kraft als (zweiter bzw. dritter) Doppel-Piezo-Aktor parallel-geschaltet (in Figuren 8a, 8c, 8d aufgrund der Ansichten in einem Querschnitt nicht erkennbar).

Wie insb. den Figuren 6, 8a (unter Bezugnahme auf die in der Aufsicht der Fig. 4a angezeichnete Ansichtsschnittlinie A-A), 8b, 8d entnehmbar, ist die zweite Klemmvorrichtung 32 mit einem schwenkarmartigen Klemmhebel 35 (als eine bevorzugte Festkörpergelenk-gelagerte Mechanik) ausgebildet. Der Klemmhebel 35 um die Längsachse M (insb. Fig. 8d) der Linearführung 60 drehbar gelagert. Das piezoelektrisch aktuierte Ausdehnen des dritten Piezo-Aktors 3 bewirkt durch dessen an dem Klemmhebel 35 angreifende Auslenkungskraft bzw. dritte Piezo-Kraft (vgl. die mittels des Pfeils oben links in Fig. 8d angedeutete Auslenkung in Drehrichtung) den zweiten Kopplungzustand. Die dritte Piezo-Kraft wird in eine, dazu Hebelgesetz-mäßig verstärkte, an der Führungsschiene 30 ansetzende Führungsschienen-Klemmkraft umgesetzt.

Wie insb. anhand der Figur 6 nachvollziehbar, ist der Schlitten 20 in einem äußeren Blechkörper angeordnet. Ein Schlittenandrückelement 29 ist als eine (oben in Fig. 6) mittels der Torx-Schrauben gesicherte bügelförmige Drahtfeder angeordnet. Das Schlittenandrückelement 29 bewirkt, dass der Blechkörper immer spielfrei zu den quer oszillierenden dritten Piezos 3 (Ausdehnen nach rechts oben in Fig. 6) verbunden ist. Wenn das Paar der dritten Piezos 3 expandiert, wird das Schlittenandrückelement 29 vorgespannt, um Gegenkräfte nach unten auszuüben. Insofern bewirkt das (bügelförmige) Schlittenandrückelement 29 in seinem Bestreben, sich geradlinig auszurichten, eine rückstellende Andruckkraft gegen den Schlitten 20 bzw. eine Gegenkraft zu der aus der Expansion der dritten Piezos 3 resultierenden dritten Piezo-Kraft. Mit anderen Worten, wirkt die Federkraft des (bügelförmigen) Schlittenandrückelements 29 nach unten bzw. gegen den Blechkörper, wodurch die der Sicherung des Schlittens 20 dienende Führungsschienen-Klemmkraft erzeugt wird.

Fig. 9 bzw. Fig. 10 zeigen ein zweites bzw. drittes Ausführungsbeispiel der offenbarungsgemäßen piezoelektrischen Antriebseinheit sowie des zugehörigen Verfahrens. Hierin wird die zeitliche Abfolge bzw. Koordination von mittels einer (mit einer gestrichelten Linie eingetragenen bzw. dargestellten) Steuereinheit 110 ausführbaren (Arbeitsverfahrens-) Schritten in einem (wiederholbaren) Grundzyklus S1 bis S4 bzw. in einem erweiterten Zyklus S101 bis S106 schematisch (bzw. prinzipiell) veranschaulicht. Dabei ist die Steuereinheit 110 eingerichtet, jeweilig den zumindest einen ersten / zweiten / ggf. dritten Piezo-Aktor (via der gestrichelt-liniert angedeuteten Steuerverbindungen) (zur piezoelektrischen Oszillation) zu aktuieren (bzw. zu betätigen, auszulösen).

Kurz gesagt, betrifft der Grundzyklus S1 bis S4 eine abwechselnde Koordination, bei der zum einen entweder der Schlitten sich bewegt oder sich nicht bewegt sowie zum anderen die Schubstange 10 von dem Schlitten geklemmt wird oder nicht geklemmt wird.

Insofern die in Fig. 10 gezeigte Abfolge den um die optionalen Schritte S102 und S104 erweiterten Zyklus betrifft, korrespondiert Fig. 10 insb. mit dem ersten Ausführungsbeispiel (vgl. Figuren 1 bis 8d) der offenbarungsgemäßen piezoelektrischen Antriebseinheit, welche zusätzlich die bevorzugt vorgesehene zweite Klemmvorrichtung (Sicherung an der Führungsschiene 30) aufweist. Aus Gründen einer übersichtlichen Darstellung sind in den Figuren 9 und 10 vereinfachend nicht die Längshub-Vorrichtung 11, die erste Klemmvorrichtung 21 und ggf. die zweite Klemmvorrichtung 32 (Bezugszeichen unter Bezugnahme auf die die strukturellen Vorrichtungsaspekte betonenden Figuren 1 bis 8d) als solche gezeigt, sondern lediglich einer des zugehörigen ersten Piezo-Aktors 1, einer des zugehörigen zweiten Piezo-Aktors 2 sowie ggf. einer des zugehörigen dritten Piezo-Aktors 3 dargestellt. Insofern ist die (relative bzw. inkrementale) Position (auf der horizontal eingezeichneten Verfahrachse) des (gleichfalls nicht dargestellten) Schlittens (20) anhand der (relativen bzw. inkrementalen) Position des zweiten Piezo-Aktors 2 (ggf. zusammen mit der des dritten Piezo-Aktors 3) zu entnehmen:
Anfänglich (bzw. zu jedem neuen Zyklusbeginn des wiederholbaren Zyklus S1 bis S4; S101 bis S106 erfolgt ein Schritt S1, S101 einer Kopplung/Klemmung des Schlittens an der Schubstange 30. Dabei wird der Schlitten an der Schubstange 30 mittels piezoelektrisch aktuierten Ausdehnen des zweiten Piezo-Aktors 2 geklemmt (siehe vertikale Pfeile in Fig. 9). Dies entspricht dem (temporären) ersten Kopplungszustand der ersten Klemmvorrichtung des Schlittens.

Kurz gesagt, wird in Schritt S1, S101 die Schubstange10 vom Schlitten gegriffen (mittels zweitem Piezo-Aktor 2 geklemmt).

Sodann erfolgt optional (nur Fig. 10) ein Schritt S102 eines Wechseln aus einem zweiten Kopplungszustand (laufhemmende Sicherung des Schlittens an der Führungsschiene 30) in einen (temporären) zweiten Entkopplungszustand (lauffreigebende Entsicherung des Schlittens von der Führungsschiene 30). Dazu wird der dritte Piezo-Aktor 3 (der zweiten Klemmvorrichtung des Schlittens) zum Ausdehnen piezoelektrisch aktuiert, so dass die zuvor bestehende Klemmung lauffreigebend aufgehoben ist.

Kurz gesagt, wird in (optionalem) Schritt S102 die Klemmung bzw. Laufhemmung des Schlittens auf der Führungsschiene 30 aufgehoben bzw. gelöst.

Sodann erfolgt ein weiterer Schritt S2, S103 (eines Erzeugens) eines Schlittenvorschubs s des Schlittens, welcher durch einen Längshub h der Schubstange ausgelöst bzw. bewirkt wird (siehe in Fig. 9/10 den horizontalen Pfeil sowie die links im Bild befindliche feingestrichelte Vertikallinie). Dazu dehnt sich der erste Piezo-Aktor 1 der Längshub-Vorrichtung von einer axialen Ausgangsposition aus. Es sei angemerkt, dass in der schematischen Darstellung der Figuren 9, 10 der erste Piezohub mit dem Längshub h zusammenfällt. D.h. eine ggf. vorzugsweise vorgesehene Hebelumsetzung (vgl. Figuren 4a bis 4d) ist hierin vernachlässigt; Analoges gilt für die Piezo-Aktoren 2, 3.

Kurz gesagt, vollführt in Schritt S2, S103 die Schubstange 10 Längshub h, indem sie um einen ersten Piezohub des ersten Piezos 1 vorgeschoben wird, und nimmt dabei den vorab geklemmten Schlitten mit.

Sodann erfolgt optional (nur Fig. 10) ein Schritt S104 eines (Zurück-)Wechseln aus dem zweiten Entkopplungszustand (lauffreigebende Entsicherung des Schlittens von der Führungsschiene 30) in den (temporären) zweiten Kopplungszustand (laufhemmende Sicherung des Schlittens an der Führungsschiene 30). Dazu wird der dritte Piezo-Aktor 3 (der zweiten Klemmvorrichtung des Schlittens) zum Zusammenziehen piezoelektrisch aktuiert. Aufgrund der Kopplung/Klemmung des Schlittens an der Führungsschiene 30 ist der Schlitten gegen Verrutschen gesichert

Kurz gesagt, wird in (optionalem) Schritt S104 der Schlitten auf der Führungsschiene 30 geklemmt bzw. gesichert bzw. laufgehemmt.

In einem nachfolgenden Schritt S3, S105 findet eine Entkopplung des Schlittens von der Schubstange 30 statt. Dazu wird der Schlitten von der Schubstange 30 mittels piezoelektrisch aktuierten Zusammenziehen des zweiten Piezo-Aktors 2 gelöst (siehe vertikale Pfeile in Fig. 9). Dies entspricht dem ersten (temporären) Entkopplungszustand der ersten Klemmvorrichtung des Schlittens.

Kurz gesagt, löst in Schritt S3, S105 der Schlitten dessen Klemmung zur Schubstange 30.

Anschließend wird in einem Schritt S4, S106 einer von dem Schlitten 10 getrennten Bewegung der Schubstange 10 diese zurück auf deren Ausgangsposition bewegt bzw. gezogen werden (siehe in Fig. 9/10 den horizontalen Pfeil sowie die links im Bild befindliche feingestrichelte Vertikallinie). Dazu zieht sich der erste Piezo-Aktor 1 der Längshub-Vorrichtung zurück auf die axialen Ausgangsposition zusammen

Kurz gesagt, wird in Schritt S4, S106 die Schubstange 10 wieder auf deren Ausgangsposition zurückgezogen, während der Schlitten an einer neuen (inkrementalen) Vorschubposition verbleibt. Man vergleiche hierzu den Versatz des Schlittens gemäß dem des zweiten Piezo-Aktors 2 zu Beginn des neuen Zyklus beim wiederholten Schritt S1 bzw. S101 (unten im Bild) gegenüber dessen Position zu Beginn des ersten Zyklus (oben im Bild).

Durch (vielfache) Wiederholung des Zyklus addieren bzw. kumulieren sich die inkrementalen Schlittenvorschübe s. Somit entsteht in Summe der Kolbenstößel-Verfahrweg X (unter Bezugnahme auf die Figuren 3a/3b).

### Liste der Bezugszeichen

- 1: erster Piezo-Aktor
- 2: zweiter Piezo-Aktor
- 3: dritter Piezo-Aktor
- 10: Schubstange
- 11: Längshub-Vorrichtung
- 15: Hebelwippenelement
- 16: Hebelwippen-Mittenabschnitt
- 21: erste Klemmvorrichtung
- 20: Schlitten
- 25: Biegefeder
- 29: Schlittenandrückelement
- 30: Führungsschiene
- 32: zweite Klemmvorrichtung
- 35: Klemmhebel
- 60: Linearführung
- 70: Antriebsrahmen
- 75: Rahmenplatte
- 90: Kolbenstößel
- 100: Antriebseinheit
- 110: Steuereinheit
- a: Hebelarm des dritten Piezos (zweite Klemmvorrichtung)
- b: Hebelarm des Klemmhebels (zweite Klemmvorrichtung)
- c: Hebelarm des zweiten Piezos (erste Klemmvorrichtung)
- d: Hebelarm der Biegefeder (erste Klemmvorrichtung)
- e: langer Hebelwippenarm (Längshub-Vorrichtung)
- f: kurzer Hebelwippenarm (Längshub-Vorrichtung)
- h: Längshub
- s: Schlittenvorschub
- x: Längsrichtung
- y: Breitenrichtung (Querrichtung)
- z: Höhenrichtung (Querrichtung)
- F2: Biederfeder-Verformungskraft
- M: Längsachse (Linearführung)
- R: Anlagepunkt (Biegefeder)
- Q: Drehpunkt (Biegefeder)
- X: Kolbenstößel-Verfahrweg
- Y: Hebelwippen-Drehachse

- S1 - S600: Verfahrensschritte

## Patentansprüche

1. Piezoelektrische Antriebseinheit (100) zum Abgeben und/oder Dosieren medizinischer Flüssigkeiten aus einem medizinischen Behälter, insbesondere für eine in einer axialen Längsrichtung (x) verfahrende medizintechnische Spritzenpumpe, Infusionspumpe oder dergleichen, mit:
- einem Antriebsrahmen (70),
- einer Steuereinheit (110),
- einer Schubstange (10), welche an dem Antriebsrahmen (70) mittels einer Längshub-Vorrichtung (11) in der Längsrichtung (x) um einen Längshub (h) oszillierend bewegbar eingerichtet ist, um bei Einwirken zumindest eines mit der Steuereinheit (110) verbundenen ersten Piezo-Aktors (1) der Längshub-Vorrichtung (11) durch dessen Ausdehnen und Zusammenziehen vor- und zurückbewegt zu werden,
- einer an dem Antriebsrahmen (70) parallel zu der Schubstange (10) angeordneten Linearführung (60),
- einem zur Förderung in der Längsrichtung (x) entlang der Linearführung (60) verfahrbaren Schlitten (20), und
- einer von der Steuereinheit (110) koordinierten ersten Klemmvorrichtung (21) des Schlittens (20), die eingerichtet ist, um zwischen einem ersten Kopplungszustand zur Kopplung des Schlittens (20) an der Schubstange (10) und einem ersten Entkopplungszustand zur Entkopplung des Schlittens (20) von der Schubstange (10) zu wechseln, wobei
der erste Kopplungszustand vorgesehen ist, um währenddessen einen Schlittenvorschub (s) des Schlittens (20) gemäß dem Längshub (h) der von einer axialen Ausgangsposition bewegbaren Schubstange auf eine in Längsrichtung (x) inkrementale Vorschubposition des Schlittens (20) zu erzeugen,
der erste Entkopplungszustand vorgesehen ist, um die Schubstange (10) für eine von dem Schlitten (20) getrennte Bewegung für deren Rückzugsbewegung zurück auf die Ausgangsposition bei gleichzeitigem Verbleib des Schlittens (20) auf der inkrementalen Vorschubposition freizugeben; und
die erste Klemmvorrichtung (21) zumindest einen zu ihrer Aktuierung mit der Steuereinheit (110) verbundenen zweiten Piezo-Aktor (2) aufweist, wobei der zumindest eine zweite Piezo-Aktor (2) eingerichtet ist, durch dessen Ausdehnen oder Zusammenziehen zumindest den ersten Kopplungszustand zu bewirken.

2. Piezoelektrische Antriebseinheit (100) nach Anspruch 1, **dadurch gekennzeichnet, dass** der zumindest eine zweite Piezo-Aktor (2) eingerichtet ist, durch dessen Ausdehnen die erste Klemmvorrichtung (21) in den ersten Entkopplungszustand freizugeben, insbesondere den ersten Entkopplungszustand zu bewirken und/oder durch dessen Zusammenziehen den ersten Kopplungszustand zu bewirken.

3. Piezoelektrische Antriebseinheit (100) nach Anspruch 1 oder 2, **dadurch gekennzeichnet, dass** der zumindest eine zweite Piezo-Aktor (2) eingerichtet ist, durch dessen Ausdehnen mittels der ersten Klemmvorrichtung (21) in einer Querrichtung (y, z) freigebend und/oder klemmend auf die Schubstange (10) zu wirken, insbesondere eine in der ersten Klemmvorrichtung (2) wirkende Druckkraft (F2) oder eine resultierende Rückstellkraft, welche einen in der Querrichtung (y, z) auf die Schubstange (10) wirkenden Vektoranteil aufweist, vorzusehen.

4. Piezoelektrische Antriebseinheit (100) nach einem der vorstehenden Ansprüche, **dadurch gekennzeichnet, dass** die piezoelektrische Antriebseinheit (100) weiterhin aufweist:
- eine parallel zu der Schubstange (10) an dem Antriebsrahmen (70) vorgesehene Führungsschiene (30); und
- eine zweite Klemmvorrichtung (32) des Schlittens (20), welche mittels des zumindest einen zweiten Piezo-Aktors (2) und/oder zumindest eines mit der Steuereinheit (110) verbundenen dritten Piezo-Aktors (3) aktuierbar ist, wobei die zweite Klemmvorrichtung (32) eingerichtet ist, um zwischen einem, insbesondere stromlos vorgesehenen, zweiten Kopplungszustand zur laufhemmenden Sicherung des Schlittens (20) an der Führungsschiene (30) gegen nicht intendierte Axialbewegungen und einem zweiten Entkopplungszustand zur lauffreigebenden Entsicherung des Schlittens (20) von der Führungsschiene (30) zu wechseln.

5. Piezoelektrische Antriebseinheit (100) nach dem direkt vorstehenden Anspruch, **dadurch gekennzeichnet, dass** die Steuereinheit (110) eingerichtet ist:
den zweiten Entkopplungszustand jeweilig zeitlich vorausgehend oder gleichzeitig zu dem ersten Kopplungszustand vorzusehen, um einen Schlittenvorschub (s) des Schlittens (20) gemäß dem Längshub (h) der von einer axialen Ausgangsposition bewegbaren Schubstange (10) auszuführen; und/oder
den zweiten Kopplungszustand jeweilig zeitlich direkt nachfolgend oder gleichzeitig zu dem ausgeführten Schlittenvorschub (s) vorzusehen.

6. Piezoelektrische Antriebseinheit (100) nach einem der vorstehenden Ansprüche, **dadurch gekennzeichnet, dass** die Längshub-Vorrichtung (11) und/oder die erste Klemmvorrichtung (21) und/oder die zweite Klemmvorrichtung (32) als eine jeweilige Festkörpergelenk-gelagerte Mechanik ausgebildet ist bzw. sind, insbesondere hinsichtlich der Oszillation zumindest eines Piezo-Aktors aus dem zumindest einen ersten, zweiten und/oder dritten Piezo-Aktor (1, 2, 3) dauerfest ausgebildet ist bzw. sind.

7. Piezoelektrische Antriebseinheit (100) nach dem direkt vorstehenden Anspruch, **dadurch gekennzeichnet, dass** im Falle der, als eine Festkörpergelenk-gelagerte Mechanik ausgebildeten, ersten Klemmvorrichtung (21) diese eine Biegefeder (25) aufweist, welche in einem Schubstangen-Klemmabschnitt (26) zwischen der Schubstange (10) und dem Schlitten (20) angeordnet ist, wobei die Biegefeder (25):
- an dem Antriebsrahmen (70) in einem Biegefeder-Drehpunkt (Q) drehbar gelagert ist, um einen hin zu dem Schubstangen-Klemmabschnitt (26) verlaufenden Biegefeder-Hebelarm (d) auszubilden;
- vorzugsweise weiterhin an dem Schlitten (20) in einem Anlagepunkt (R) in Verlängerung des Biegefeder-Hebelarms (d) rückstellend gegengelagert ist; und
- ausgebildet ist, um sich bei piezoelektrisch aktuiertem Ausdehnen des zweiten Piezo-Aktors (2) in der Querrichtung (y, z) derart zu verformen, dass die Schubstange (10) von dem Schlitten (20) in den ersten Entkopplungszustand freigegeben wird; weiter bevorzugt
- ausgebildet ist, um bei deren Verformung eine Vorspannung mit einer in der Querrichtung (y, z) resultierenden Biegefeder-Rückstellkraft (- F2) zu bewirken, dass bei Zusammenziehen des zweiten Piezo-Aktors (2) der Schlitten (20) zurück an die Schubstange (10) in den ersten Kopplungszustand koppelt.

8. Piezoelektrische Antriebseinheit (100) nach Anspruch 6 oder 7, **dadurch gekennzeichnet, dass** im Falle der, als eine Festkörpergelenk-gelagerte Mechanik ausgebildeten, zweiten Klemmvorrichtung (32) diese einen schwenkarmartigen Klemmhebel (35) aufweist, wobei der Klemmhebel (35):
- um die Längsachse (M) der Linearführung (60) drehbar gelagert ist, um bei piezoelektrisch aktuiertem Ausdehnen des zumindest einen zweiten Piezo-Aktors (2) und/oder dritten Piezo-Aktors (3) durch dessen Auslenkungskraft den zweiten Kopplungszustand zu bewirken; weiter bevorzugt
- ausgebildet ist, um eine an dem Klemmhebel (35) angreifende Auslenkungskraft des zumindest einen zweiten Piezo-Aktors (2) und/oder dritten Piezo-Aktors (3) in eine, dazu Hebelgesetz-mäßig verstärkte, an der Führungsschiene (30) ansetzende Führungsschienen-Klemmkraft zu übersetzen.

9. Piezoelektrische Antriebseinheit (100) nach einem der Ansprüche 6 bis 8, **dadurch gekennzeichnet, dass** im Falle der, als eine Festkörpergelenk-gelagerte Mechanik ausgebildeten, Längshub-Vorrichtung (11) diese ein zweiseitiges Hebelwippenelement (15) aufweist, wobei das Hebelwippenelement (15):
- in dessen zentralen Hebelwippen-Mittenabschnitt (16) an einem hinsichtlich einer Richtung des Schlittenvorschubs (s) rückwärtigen Ende des Antriebsrahmen (70), insbesondere an einer hinteren Rahmenplatte (75), um eine zur Längsrichtung (x) orthogonale Hebelwippen-Drehachse (Y) schwenkbar gelagert ist und dazu zweiseitig an den gegenüberliegenden Enden jeweilig einen kurzen Hebelwippenarm (f) und einen langen Hebelwippenarm (e) ausbildet, um bei piezoelektrisch aktuiertem Ausdehnen des zumindest einen ersten Piezo-Aktors (1) um einen ersten Piezohub den Längshub (h) der Schubstange (10) zu bewirken; weiter bevorzugt
- ausgebildet ist, um den an dem kurzen Hebelwippenarm (f) ansetzenden ersten Piezohub des ersten Piezo-Aktors (1) in den, dazu Hebelgesetz-mäßig vergrößerten, Längshub (h) der an dem langen Hebelwippenarm (e) angeordneten Schubstange (10) zu übersetzen.

10. Verfahren zum Erzeugen eines Schlittenvorschubs des Schlittens einer piezoelektrischen Antriebseinheit (100) nach einem der vorstehenden Ansprüche mit den über die Steuereinheit (110), insbesondere in Weise einer wiederholbaren Schrittsequenz (S1-S4; S101-S106), nachfolgend ausführbaren Schritten:
- Kopplung (S1, S101) des Schlittens (20) an der Schubstange (10) mittels der ersten Klemmvorrichtung (21) des Schlittens (20) in einen piezoelektrisch aktuierten ersten Kopplungszustand;
- vorzugsweise optionales Wechseln (S102) mittels der gegebenenfalls vorgesehenen zweiten Klemmvorrichtung (32) des Schlittens (20) aus einem zweiten Kopplungszustand zur laufhemmenden Sicherung des Schlittens (20) an der gegebenenfalls vorgesehenen Führungsschiene (30) in einen piezoelektrisch aktuierten zweiten Entkopplungszustand zur lauffreigebenden Entsicherung des Schlittens (20) von der Führungsschiene (30);
- Erzeugen (S2, S103) eines Schlittenvorschubs des Schlittens (20) auf eine in Längsrichtung (x) inkrementale Vorschubposition, gekoppelt mit einem Längshub (h) der Schubstange (10) von einer axialen Ausgangsposition bei mittels des zumindest einen ersten Piezo-Aktors (1) piezoelektrisch aktuiertem Einwirken der Längshub-Vorrichtung (11);
- vorzugsweise optionales Wechseln (S104) mittels der gegebenenfalls vorgesehenen zweiten Klemmvorrichtung (32) des Schlittens (20) aus dem zweiten Entkopplungszustand zurück in den piezoelektrisch aktuierten zweiten Kopplungszustand zur laufhemmenden Sicherung des Schlittens (20) an der gegebenenfalls vorgesehenen Führungsschiene (30), insbesondere auf der inkrementalen Vorschubposition;
- Entkopplung (S3, S105) des Schlittens (20) von der Schubstange (10) mittels der ersten Klemmvorrichtung (21) des Schlittens (20) in einen piezoelektrisch aktuierten ersten Entkopplungszustand;
- getrennte Bewegung (S4, S106) der Schubstange (10) als eine Rückzugsbewegung der Schubstange (10) von der in der Längsrichtung (x) inkrementalen Vorschubposition zurück auf die Ausgangsposition, bei mittels des zumindest einen ersten Piezo-Aktors (1) piezoelektrisch aktuiertem Einwirken der Längshub-Vorrichtung (11).

11. Verfahren nach dem direkt vorstehenden Anspruch, **dadurch gekennzeichnet, dass** weiterer Schritt über die Steuereinheit (110) ausführbar ist:
- Steuern, vorzugsweise stufenloses Steuern, der Oszillation zumindest eines Piezo-Aktors (1, 2, 3) aus dem zumindest einen, zweiten und/oder dritten Piezo-Aktor, insbesondere Steuern des Längshubs (h) der an dem Antriebsrahmen (70) mittels der Längshub-Vorrichtung (11) oszillierend bewegbaren Schubstange (10).

12. Medizintechnische Spritzenpumpe mit einer piezoelektrischen Antriebseinheit (100) nach einem der vorstehenden auf die Antriebseinheit (100) gerichteten Ansprüche, insbesondere mobil von einem Anwender tragbare Infusionspumpe.

13. Computerprogramm auf einem Datenträger, eingerichtet zur Durchführung des Verfahrens nach einem der Ansprüche 10 oder 11, wobei das Verfahren die über die Steuereinheit (110) ausführbaren Schritte, insbesondere die wiederholbare Schrittsequenz (S1-S4; S101-S106), umfasst.

## Claims

1. A piezoelectric drive unit (100) for dispensing and/or dosing of medical liquids from a medical container, especially for a medical-technical syringe pump, infusion pump or the like travelling in an axial longitudinal direction (x), comprising:
- a drive frame (70),
- a control unit (110),
- a push rod (10) which is configured at the drive frame (70) to be movable in an oscillating manner about a longitudinal stroke (h) in the longitudinal direction (x) by means of a longitudinal stroke device (11) in order to be moved back and forth with acting of at least one first piezo actuator (1) of the longitudinal stroke device (11) connected to the control unit (110) by expansion and contraction thereof,
- a linear guide (60) arranged at the drive frame (70) parallel to the push rod (10),
- a carriage (20) movable along the linear guide (60) for conveying in the longitudinal direction (x), and
- a first clamping device (21) of the carriage (20), coordinated by the control unit (110), which is configured to switch between a first coupling state for coupling of the carriage (20) to the push rod (10) and a first decoupling state for decoupling of the carriage (20) from the push rod (10),
wherein
the first coupling state is provided to meanwhile generate a carriage advance (s) of the carriage (20) according to the longitudinal stroke (h) of the push rod movable from an axial starting position to an advance position of the carriage (20) incremental in longitudinal direction (x),
the first decoupling state is provided to release the push rod (10) for a motion separate from the carriage (20) for its retracting motion back to the starting position while remaining of the carriage (20) at the incremental advance position, and
the first clamping device (21) comprises at least one second piezo actuator (2) connected, for its actuating, to the control unit (110), whereby the at least one second piezo actuator (2) is configured to cause, by expansion or contraction thereof, at least the first coupling state.

2. The piezoelectric drive unit (100) according to claim 1, **characterized in that** the at least one second piezo actuator (2) is configured to release, by expansion thereof, the first clamping device (21) into the first decoupling state, especially to cause the first decoupling state and/or to cause, by contraction thereof, the first coupling state.

3. The piezoelectric drive unit (100) according to claim 1 or 2, **characterized in that** the at least one second piezo actuator (2) is configured to act, by expansion thereof, by means of the first clamping device (21) in a cross direction (y, z) in a releasing and/or clamping manner on the push rod (10), especially to provide a compressive force (F2) acting in the first clamping device (2) or a resulting restoring force having a vector component acting in the cross direction (y, z) on the push rod (10).

4. The piezoelectric drive unit (100) according to any of the preceding claims, **characterized in that** the piezoelectric drive unit (100) further comprises:
- a guide rail (30) provided parallel to the push rod (10) at the drive frame (70); and
- a second clamping device (32) of the carriage (20), which is actuatable by means of the at least one second piezo actuator (2) and/or of at least one third piezo actuator (3) connected to the control unit (110), whereby the second clamping device (32) is configured to switch between a second coupling state, especially provided without current, for run-inhibiting securing of the carriage (20) to the guide rail (30) against unintended axial movements and a second decoupling state for run-free de-securing of the carriage (20) from the guide rail (30).

5. The piezoelectric drive unit (100) according to the directly preceding claim, **characterized in that** the control unit (110) is configured:
to provide the second decoupling state, respectively, temporally precedingly or simultaneously to the first coupling state in order to perform a carriage advance (s) of the carriage (20) according to the longitudinal stroke (h) of the push rod (10) movable from an axial starting position; and/or
to provide the second coupling state, respectively, temporally directly subsequently or simultaneously to the performed carriage advance (s).

6. The piezoelectric drive unit (100) according to any of the preceding claims, **characterized in that** the longitudinal stroke device (11) and/or the first clamping device (21) and/or the second clamping device (32) is or, respectively, are formed as a respective solid body joint-supported mechanism, especially is or, respectively, are formed durable with respect to the oscillation of at least one piezo actuator from the at least one first, second and/or third piezo actuator (1, 2, 3).

7. The piezoelectric drive unit (100) according to the directly preceding claim, **characterized in that** in the case of the first clamping device (21) formed as a solid body joint-supported mechanism, it comprises a bending spring (25) which is arranged in a push rod clamping section (26) between the push rod (10) and the carriage (20), whereby the bending spring (25):
- is rotatably supported on the drive frame (70) in a bending spring pivot point (Q) to form a bending spring lever arm (d) extending toward the push rod clamping section (26);
- preferably is further restoringly counter-supported on the carriage (20) in an abutment point (R) in extension of the bending spring lever arm (d); and
- is formed to deform with piezoelectrically actuated expansion of the second piezo actuator (2) in the cross direction (y, z) such that the push rod (10) is released from the carriage (20) into the first decoupling state; further preferably
- is formed to cause, with deformation thereof, a preload with a bending spring restoring force (- F2) resulting in the cross direction (y, z), so that, with contraction of the second piezo actuator (2), the carriage (20) couples back to the push rod (10) into the first coupling state.

8. The piezoelectric drive unit (100) according to claim 6 or 7, **characterized in that** in the case of the second clamping device (32) formed as a solid body joint-supported mechanism, it comprises a pivot arm-like clamping lever (35), whereby the clamping lever (35):
- is rotatably supported about the longitudinal axis (M) of the linear guide (60) in order to cause the second coupling state with piezoelectrically actuated expansion of the at least one second piezo actuator (2) and/or third piezo actuator (3) by its deflection force; further preferably
- is formed to translate a deflection force of the at least one second piezo actuator (2) and/or third piezo actuator (3) acting on the clamping lever (35) into a guide rail clamping force, thereto amplified according to the lever law, applied to the guide rail (30).

9. The piezoelectric drive unit (100) according to any of claims 6 to 8, **characterized in that** in the case of the longitudinal stroke device (11) formed as a solid body joint-supported mechanism, it comprises a double-sided lever rocker element (15), whereby the lever rocker element (15):
- in its central lever rocker midsection (16) at a rear end of the drive frame (70) with respect to a direction of the carriage advance (s), especially at a rear frame plate (75), is pivotally supported about a lever rocker rotation axis (Y) orthogonal to the longitudinal direction (x) and for this purpose forms on both sides at the opposite ends a short lever rocker arm (f) and a long lever rocker arm (e), respectively, in order to cause the longitudinal stroke (h) of the push rod (10) with piezoelectrically actuated expansion of the at least one first piezo actuator (1) by a first piezo stroke; further preferably
- is formed to translate the first piezo stroke of the first piezo actuator (1) applied to the short lever rocker arm (f) into the longitudinal stroke (h) of the push rod (10) arranged on the long lever rocker arm (e), which is enlarged according to the lever law.

10. A method for generating a carriage advance of the carriage of a piezoelectric drive unit (100) according to any of the preceding claims comprising the steps subsequently executable via the control unit (110), especially in the manner of a repeatable step sequence (S1-S4; S101-S106):
- coupling (S1, S101) of the carriage (20) to the push rod (10) by means of the first clamping device (21) of the carriage (20) into a piezoelectrically actuated first coupling state;
- preferably optional switching (S102) by means of the, if applicable, provided second clamping device (32) of the carriage (20) from a second coupling state for run-inhibiting securing of the carriage (20) at the, if applicable, provided guide rail (30) into a piezoelectrically actuated second decoupling state for run-free de-securing of the carriage (20) from the guide rail (30);
- generating (S2, S103) of a carriage advance of the carriage (20) to an advance position incremental in longitudinal direction (x), coupled with a longitudinal stroke (h) of the push rod (10) from an axial starting position with acting of the longitudinal stroke device (11) piezoelectrically actuated by means of the at least one first piezo actuator (1);
- preferably optional switching (S104) by means of the, if applicable, provided second clamping device (32) of the carriage (20) from the second decoupling state back into the piezoelectrically actuated second coupling state for run-inhibiting securing of the carriage (20) to the, if applicable, provided guide rail (30), especially on the incremental advance position;
- decoupling (S3, S105) of the carriage (20) from the push rod (10) by means of the first clamping device (21) of the carriage (20) into a piezoelectrically actuated first decoupling state;
- separate motion (S4, S106) of the push rod (10) as a retracting motion of the push rod (10) from the advance position incremental in the longitudinal direction (x) back to the starting position, with acting of the longitudinal stroke device (11) piezoelectrically actuated by means of the at least one first piezo actuator (1).

11. The method according to the directly preceding claim, **characterized in that** further step is executable via the control unit (110):
- controlling, preferably stepless controlling, of the oscillation of at least one piezo actuator (1, 2, 3) from the at least one, second and/or third piezo actuator, especially controlling of the longitudinal stroke (h) of the push rod (10) movable in an oscillating manner at the drive frame (70) by means of the longitudinal stroke device (11).

12. A medical-technical syringe pump with a piezoelectric drive unit (100) according to any of the preceding claims directed to the drive unit (100), in particular a fusion pump portable by a user.

13. A computer program on a data medium, configured for performing the method according to any of claims 10 or 11, whereby the method comprises the steps executable via the control unit (110), especially the repeatable step sequence (S1-S4; S101-S106).

## Revendications

1. Unité d'entraînement (100) piézo-électrique pour la distribution et/ou le dosage de liquides médicaux à partir d'un récipient médical, en particulier pour une pompe à seringue, pompe à perfusion médicale ou similaire se déplaçant dans un sens longitudinal (x) axial avec :
- un cadre d'entraînement (70),
- un dispositif de commande (110),
- une tige de poussée (10) qui est conçue de manière mobile en oscillation selon une course longitudinale (h) au niveau du cadre d'entraînement (70) au moyen d'un dispositif à course longitudinale (11) dans le sens longitudinal (x) afin d'être déplacée vers l'avant et l'arrière par son expansion et sa contraction lors de l'action au moins d'un premier actionneur piézo-électrique (1) du dispositif à course longitudinale (11) relié au dispositif de commande (110),
- un guidage linéaire (60) agencé au niveau du cadre d'entraînement (70) parallèlement à la tige de poussée (10),
- un chariot (20) déplaçable pour le transport dans le sens longitudinal (x) le long du guidage linéaire (60) et
- un premier dispositif de serrage (21) du chariot (20) coordonné par le dispositif de commande (110), chariot qui est conçu afin de passer d'un premier état de couplage pour le couplage du chariot (20) à la tige de poussée (10) à un premier état de découplage pour le découplage du chariot (20) de la tige de poussée (10), dans lequel
le premier état de découplage est prévu afin de générer entre-temps une avance de chariot (s) du chariot (20) selon la course longitudinale (h) de la tige de poussée mobile depuis une position de départ axiale à une position d'avance incrémentale du chariot (20) dans le sens longitudinal (x),
le premier état de découplage est prévu afin de libérer la tige de poussée (10) pour un mouvement séparé du chariot (20) pour son mouvement de retrait à la position de départ en cas de maintien simultané du chariot (20) à la position d'avance incrémentale ; et
le premier dispositif de serrage (21) présente au moins un deuxième actionneur piézo-électrique (2) relié pour son actionnement au dispositif de commande (110), dans lequel le au moins deuxième actionneur piézo-électrique (2) est conçu afin de provoquer au moins le premier état de couplage par son expansion ou sa contraction.

2. Unité d'entraînement (100) piézo-électrique selon la revendication 1, **caractérisée en ce que** le au moins deuxième actionneur piézo-électrique (2) est conçu afin de libérer par son expansion le premier dispositif de serrage (21) dans le premier état de découplage, en particulier le premier état de découplage et/ou par sa contraction le premier état de couplage.

3. Unité d'entraînement (100) piézo-électrique selon la revendication 1 ou 2, **caractérisée en ce que** le au moins deuxième actionneur piézo-électrique (2) est conçu afin d'agir par libération et/ou serrage sur la tige de poussée (10) par son expansion au moyen du premier dispositif de serrage (21) dans un sens transversal (y, z), en particulier de prévoir une force de pression (F2) agissant dans le premier dispositif de serrage (2) ou une force de rappel résultante qui présente une part de vecteur agissant dans le sens transversal (y, z) sur la tige de poussée (10).

4. Unité d'entraînement (100) piézo-électrique selon l'une quelconque des revendications précédentes, **caractérisée en ce que** l'unité d'entraînement (100) piézo-électrique présente en outre :
- un rail de guidage (30) prévu parallèlement à la tige de poussée (10) au niveau du cadre d'entraînement (70) ; et
- un second dispositif de serrage (32) du chariot (20) qui peut être actionné au moyen de l'au moins un deuxième actionneur piézo-électrique (2) et/ou au moins d'un troisième actionneur piézo-électrique (3) relié au dispositif de commande (110), dans laquelle le second dispositif de serrage (32) est conçu afin de passer d'un second état de couplage prévu en particulier sans courant pour la fixation empêchant la course du chariot (20) au rail de guidage (30) contre des mouvements axiaux non intentionnels à un second état de découplage pour le déverrouillage du rail de guidage (30) libérant la course du chariot (20).

5. Unité d'entraînement (100) piézo-électrique selon la revendication directement précédente, **caractérisée en ce que** le dispositif de commande (110) est conçu :
afin de prévoir le second état de découplage respectivement avant ou simultanément au premier état de couplage afin de réaliser une avance (s) du chariot (20) selon la course longitudinale (h) de la tige de poussée (10) mobile depuis une position de départ axiale ; et/ou
afin de prévoir le second état de couplage respectivement directement après ou simultanément à l'avance de chariot (s) réalisée.

6. Unité d'entraînement (100) piézo-électrique selon l'une quelconque des revendications précédentes, **caractérisée en ce que** le dispositif à course longitudinale (11) et/ou le premier dispositif de serrage (21) et/ou le second dispositif de serrage (32) est ou sont configurés comme une mécanique logée dans l'articulation de corps solide respective, en particulier en ce qui concerne l'oscillation au moins d'un actionneur piézo-électrique à partir duquel au moins un premier, deuxième et/ou troisième actionneur piézoélectrique (1, 2, 3) est/sont conçu(s) pour être fixé(s) de manière permanente.

7. Unité d'entraînement (100) piézo-électrique selon la revendication directement précédente, **caractérisée en ce que** dans le cas du premier dispositif de serrage (21) configuré comme une mécanique logée dans l'articulation de corps solide, celui-ci présente un ressort de flexion (25) qui est agencé dans une section de serrage de tige de poussée (26) entre la tige de poussée (10) et le chariot (20), dans laquelle le ressort de flexion (25) :
- est logé de manière rotative au niveau du cadre d'entraînement (70) dans un point de rotation de ressort de flexion afin de configurer un bras de levier de ressort de flexion (d) s'étendant jusqu'à la section de serrage de tige de poussée (26) ;
- est toujours contre-logé par rappel de préférence au niveau du chariot (20) dans un point d'appui (R) en prolongement du bras de levier de ressort de flexion (d) ; et
- est configuré afin de se déformer lors de l'expansion actionnée piézo-électriquement du deuxième actionneur piézo-électrique (2) dans le sens transversal (y, z), de telle manière que la tige de poussée (10) soit libérée du chariot (20) dans le premier état de découplage ; en outre de préférence
- est configuré afin de provoquer lors de sa déformation une précontrainte avec une force de rappel de ressort de flexion (- F2) résultante dans le sens transversal (y, z), **en ce que** lors de la contraction du deuxième actionneur piézo-électrique (2), le chariot (20) se couple à nouveau à la tige de poussée (10) dans le premier état de couplage.

8. Unité d'entraînement (100) piézo-électrique selon la revendication 6 ou 7, **caractérisée en ce que** dans le cas du second dispositif de serrage (32) configuré comme une mécanique logée dans l'articulation de corps solide, celui-ci présente un levier de serrage (35) de type bras de pivotement, dans laquelle le levier de serrage (35) :
- est logé de manière rotative autour de l'axe longitudinal (M) du guidage linéaire (60) afin de provoquer le second état de couplage lors de l'expansion actionnée piézo-électriquement de l'au moins un deuxième actionneur piézo-électrique (2) et/ou troisième actionneur piézo-électrique (3) par sa force de déviation ; en outre de préférence
- est configuré afin de traduire une force de déviation de l'au moins un deuxième actionneur piézo-électrique (2) et/ou troisième actionneur piézo-électrique (3) agissant sur le levier de serrage (35) en une force de serrage de rail de guidage agissant sur le rail de guidage (30) qui est amplifiée selon le principe du levier.

9. Unité d'entraînement (100) piézo-électrique selon la revendication 6 à 8, **caractérisée en ce que** dans le cas du dispositif à course longitudinale (11) configuré comme une mécanique logée dans l'articulation de corps solide, celui-ci présente un élément de bascule de levier (15) bilatéral, dans laquelle l'élément de bascule de levier (15) :
- est logé de manière pivotante dans sa section médiane de bascule de levier (16) centrale, au niveau d'une extrémité arrière du cadre d'entraînement (70) par rapport à un sens de l'avance de chariot (s), en particulier au niveau d'une plaque de cadre (75) arrière, autour d'un axe de rotation de bascule de levier (Y) orthogonal au sens longitudinal (x) et forme en outre des deux côtés aux extrémités opposées respectivement un bras de bascule de levier (f) court et un bras de bascule de levier (e) long afin de provoquer la course longitudinale (h) de la tige de poussée (10) lors de l'expansion actionnée piézo-électriquement de l'au moins un premier actionneur piézo-électrique (1) selon une première course piézo-électrique ; en outre de préférence
- est configuré afin de traduire la première course piézo-électrique du premier actionneur piézo-électrique (1)agissant sur le bras de bascule de levier (f) court dans la course longitudinale (h) de la tige de poussée (10) agencée sur le bras de bascule de levier (e) long qui est agrandie selon le principe du levier.

10. Procédé de génération d'une avance de chariot du chariot d'une unité d'entraînement (100) piézo-électrique selon l'une quelconque des revendications précédentes avec les étapes suivantes réalisables par le biais du dispositif de commande (110), en particulier à la manière d'une séquence d'étapes pouvant être répétée (S1- S4 ; 5101-5106) :
- le couplage (S1, S101) du chariot (20) à la tige de poussée (10) au moyen du premier dispositif de serrage (21) du chariot (20) dans un premier état de couplage actionné piézo-électriquement ;
- de préférence le changement optionnel (S102) au moyen du second dispositif de serrage (32) du chariot (20) prévu le cas échéant d'un second état de découplage pour la fixation empêchant la course du chariot (20) au rail de guidage (30) prévu le cas échéant dans un second état de découplage actionné piézo-électriquement pour le déverrouillage libérant la course du chariot (20) du rail de guidage (30) ;
- la génération (S2, S103) d'une avance de chariot du chariot (20) à une position d'avance incrémentale dans le sens longitudinal (x), couplée avec une course longitudinale (h) de la tige de poussée (10) depuis une position de départ axiale lors de l'action actionnée piézo-électriquement du dispositif à course longitudinale au moyen de l'au moins un premier actionneur piézo-électrique (1) (11) ;
- de préférence le passage optionnel (5104) au moyen du second dispositif de serrage (32) du chariot (20) prévu le cas échéant du second état de découplage dans le second état de couplage actionné piézo-électriquement pour la fixation empêchant la course du chariot (20) au rail de guidage (30) prévu le cas échéant, en particulier à la position d'avance incrémentielle ;
- le découplage (S3, S105) du chariot (20) de la tige de poussée (10) au moyen du premier dispositif de serrage (21) du chariot (20) dans un premier état de découplage actionné piézo-électriquement ;
- le mouvement séparé (S4, S106) de la tige de poussée (10) comme un mouvement de retrait de la tige de poussée (10) depuis la position d'avance incrémentale dans le sens longitudinal (x) dans la position de départ, lors de l'action actionnée piézo-électriquement du dispositif à course longitudinale (11) au moyen de l'au moins un premier actionneur piézo-électrique (1).

11. Procédé selon la revendication directement précédente, **caractérisé en ce qu'**une autre étape peut être réalisée par le biais du dispositif de commande (110) :
- la commande, de préférence la commande continue, de l'oscillation d'au moins un actionneur piézo-électrique (1, 2, 3) à partir au moins du premier, deuxième et/ou troisième actionneur piézo-électrique, en particulier la commande de la course longitudinale (h) de la tige de poussée (10) mobile en oscillation au niveau du cadre d'entraînement (70) au moyen du dispositif à course longitudinale (11).

12. Pompe à seringue médicale avec une unité d'entraînement (100) piézo-électrique selon l'une quelconque des revendications précédentes traitant de l'unité d'entraînement (100), en particulier une pompe de perfusion portable de manière mobile par un utilisateur.

13. Programme informatique sur un support de données conçu pour la réalisation du procédé selon l'une quelconque des revendications 10 ou 11, dans lequel le procédé comprend les étapes réalisables par le biais du dispositif de commande (110), en particulier la séquence d'étapes pouvant être répétée (S1-S4 ; S101-S106).
